# EUROPEAN PATENT SPECIFICATION

(11) **EP 4 566 591 B1**
(45) Date of publication and mention of the grant of the patent: **31.12.2025**
(21) Application number: 23214816.3
(22) Date of filing: 07.12.2023
(51) Int. Cl.: A61K 9/20, A61K 9/16, A61K 45/06, A61K 31/397, A61K 31/47, A61P 3/06

(54) **SOLID PHARMACEUTICAL DOSAGE FORM COMPRISING EZETIMIBE AND PITAVASTATIN**
FESTE PHARMAZEUTISCHE DARREICHUNGSFORM MIT EZETIMIBE UND PITAVASTATIN
FORME PHARMACEUTIQUE SOLIDE COMPRENANT DE L'ÉZÉTIMIBE ET DE LA PITAVASTATINE

(43) Date of publication of application: 11.06.2025
(73) Proprietor: KRKA, d.d., Novo mesto, 8501 Novo mesto (SI)
(72) Inventor: BAJC, Grega, 8501 Novo mesto (SI); DEBELJAK SIMONCIC, Kristina, 8501 Novo mesto (SI); OMERZU, Maja, 8501 Novo mesto (SI); KOSMAC, Ina, 8501 Novo mesto (SI); KROSELJ, Vesna, 8501 Novo mesto (SI)
(74) Representative: Kutzenberger Wolff & Partner

(56) References cited:
- CN-A- 112 999 178
- JP-A- 2021 008 432
- US-A1- 2022 047 547

## Description

The invention relates to solid pharmaceutical compositions comprising or essentially consisting of (A) an intragranular phase comprising or essentially consisting of (i) ezetimibe, a pharmaceutically acceptable salt and/or solvate thereof; (ii) one or more intragranular diluents; (iii) one or more intragranular binders; (iv) optionally, one or more intragranular disintegrants; and (v) optionally, one or more intragranular surfactants; and (B) an extragranular phase comprising or essentially consisting of (vi) pitavastatin, a pharmaceutically acceptable salt and/or solvate thereof; (vii) one or more extragranular diluents; (viii) optionally, one or more extragranular disintegrants; and (ix) optionally, one or more extragranular lubricants. The invention further relates to an oral dosage form comprising the solid pharmaceutical composition suitable for oral administration.

Dyslipidemia is one of the most important risk factors for cardiovascular disease (Stehbens WE. Coronary heart disease, hypercholesterolemia, and atherosclerosis. II. Misrepresented data. Exp Mol Pathol. 2001; 70: 120-139). In patients with high or very high risk of coronary heart disease (CHD) and similar critical conditions, low-density lipoprotein cholesterol (LDL-C) should be reduced by at least 30-50% in order to obtain a clinical benefit (Whayne TF., Jr. Assessment of low-density lipoprotein targets. Angiology. 2013; 64:411-416). However, it is difficult to achieve these target levels with the administration of a statin alone (Weng TC, Yang YH, Lin SJ, Tai SH. A systematic review and meta-analysis on the therapeutic equivalence of statins. J Clin Pharm Ther. 2010;35: 139-151).

Clinical studies showed that the combination of statins with a cholesterol absorption inhibitor (ezetimibe) significantly decreased LDL-C levels in patients with hypercholesterolemia, and resulted in superior lipid-lowering efficacy compared to treatment with a statin alone (Toth PP, Foody JM, Tomassini JE, et al. Therapeutic practice patterns related to statin potency and ezetimibe/simvastatin combination therapies in lowering LDL-C in patients with high-risk cardiovascular disease. J Clin Lipidol. 2014;8: 107-116 and Foody JM, Toth PP, Tomassini JE, et al. Changes in LDL-C levels and goal attainment associated with addition of ezetimibe to simvastatin, atorvastatin, or rosuvastatin compared with titrating statin monotherapy. Vase Health Risk Manag. 2013; 9:719-727). The pitavastatin/ezetimibe fixed-dose combination showed an excellent LDL-C-reducing effect by the complementary pharmacological action of each component, and its safety profile was similar to that of pitavastatin monotherapy (Tsujita K, Yokote K, Ako J, Tanigawa R, Tajima S, Suganami H in Efficacy and Safety of Pitavastatin/Ezetimibe Fixed-Dose Combination vs. Pitavastatin. J Atheroscler Thromb. 2023 Nov 1;30(11):1580-1600.

Ezetimibe with chemical name (3R,4S)-1-(4-fluorophenyl)-3-[(3S)-3-(4-fluorophenyl)-3-hydroxypropyl]-4-(4-hydroxyphenyl)azetidin-2-one is a potent cholesterol absorption inhibitor and is commercially available in the form of tablet under the tradename Ezetrol^{®}. It is indicated as adjunctive therapy to diet for use in patients with primary (heterozygous familial and non-familial) hypercholester-olaemia in whom a statin is considered inappropriate or is not tolerated. Ezetrol^{®} is indicated to reduce the risk of cardiovascular events in patients with coronary heart disease and a history of acute coronary syndrome when added to ongoing statin therapy or initiated concomitantly with a statin. Ezetrol^{®} is also indicated as adjunctive therapy to diet for use in patients with homozygous familial sitosterolemia. The product contains 10 mg ezetimibe. Ezetimibe is poorly water soluble BCS class II drug, it has poor bioavailability (35-65%) due to its low dissolution profile in gastro intestinal tract. Ezetimibe was first disclosed in EP 0 720 599.

Pitavastatin with chemical (E,3R,5S)-7-[2-cyclopropyl-4-(4-fluorophenyl) quinolin-3-yl]-3,5-dihydroxyhept-6-enoic acid is an inhibitor of the enzyme 3 -hydroxy-3 -methyl glutaryl coenzyme A reductase (HMG-CoA reductase) and is commercially available formulated as hemi calcium salt under the tradename Livalo^{®}. The product contains 1 mg, 2 mg or 4 mg pitavastatin. Pitavastatin is used together with a proper diet to lower high cholesterol levels and triglyceride (fat) levels in the blood and increase good cholesterol (HDL) in patients with primary hyperlipidemia or mixed dyslipidemia. This medicine may help prevent medical problems (e.g. heart attacks, strokes) that are caused by fat clogging the blood vessels. Livalo^{®} is also used to treat heterozygous familial hypercholesterolemia in children 8 years of age and older. Pitavastatin was first disclosed in EP 0 304 063.

Combination compositions comprising a cholesterol absorption inhibitor ezetimibe in combination with pitavastatin are already known.

WO 2006 025378 A1 relates to a therapeutic agent for hyperlipemia or hypercholesterolemia, which comprises administering effective doses of ezetimibe and pitavastatin or a salt or lactone derivative thereof. Pitavastatin calcium salt and ezetimibe are suspended in a 0.5% by mass aqueous solution of sodium carboxymethylcellulose.

JP 2019 043880 A, WO 2020 178878 A1 and JP 2021 008432 A relate to a pharmaceutical composition containing pitavastatin or a salt or a solvate thereof; and ezetimibe or a salt or a solvate thereof. Exemplified are compositions wherein both active ingredients are either incorporated into in a single granule or into separate granules. It is stated that the increase in the degradation product derived from pitavastatin can be suppressed by allowing pitavastatin or a salt thereof or a solvate thereof to coexist with ezetimibe or a salt thereof or a solvate thereof.

CN 112999178 A relates to an ezetimibe pitavastatin calcium compound double-layer tablet, which comprises active ingredients of ezetimibe and pitavastatin calcium, wherein the two active ingredients are respectively granulated with other auxiliary materials, uniformly mixed and pressed into double-layer tablets.

US 2022 0047547 A1 relates to a solid oral pharmaceutical composition comprising pitavastatin or a pharmaceutically acceptable salt thereof; ezetimibe or a pharmaceutically acceptable salt thereof; and at least one excipient, including a diluent, a stabilizing agent, a disintegrant, a binding agent, a sweetener, a lubricant, a glidant, a flavor, a coloring agent or a combination thereof. Exemplified are single-layered tablets with a common granulate, double-layered tablets with separate granulates, capsules filled with separate mini-tablets or separate granules, or pellets comprising both active ingredients separated by a protection layer. Adequate stability cannot be achieved by using a single-layered tablet. Furthermore, in case of single-layered and double-layered tablets, optimal *in vitro* dissolution profile cannot be achieved.

The properties of the known combination products of ezetimibe and pitavastatin are not satisfactory in every respect. While pitavastatin has been reported to be more stable in near neutral to alkaline pH, such conditions are detrimental to the stability of ezetimibe. There is a demand for improved oral dosage forms that comprise fixed doses of ezetimibe and pitavastatin and that are effective in the treatment of abnormal lipid levels. The dosage forms should combine pharmacologic efficacy with adequate physical and chemical stability as well as dissolution profile of both ezetimibe and pitavastatin. Further, there is a demand for a reliable, economical, simple and robust method for the manufacture of such dosage forms. When more than one active substance is formulated in a combination product, it is usually required for regulatory reasons, particularly with respect to bioequivalence, that the dissolution profile of each active ingredient within the combination is similar to the dissolution profile of the two reference products that contain only one of the two active ingredients. In the present case, the reference product only containing ezetimibe is Ezetrol^{®}, the reference product only containing pitavastatin is Livalo^{®}.

A major challenge in achieving the desired dissolution profile of a new combination product is the low solubility of ezetimibe. Ezetimibe is classified in class II of the Biopharmaceutics Classification System (BCS), which means that it has low solubility and high permeability. This means that *in vivo* drug dissolution is a rate limiting step for absorption, thus in order to achieve desired bioavailability the drug should dissolve quickly, meaning that typically 55% of ezetimibe are dissolved within 5 minutes, and 90% of ezetimibe are dissolved within 10 minutes under *in vitro* conditions.

It is an object of the invention to provide a solid oral dosage form comprising ezetimibe and pitavastatin and showing at least a similar *in vitro* dissolution profile compared to that of Ezetrol^{®} and Livalo^{®}. The solid dosage form should be stable. Preparation of the solid dosage form should be possible by a conventional, reliable, economical, simple and robust manufacturing process.

Further, it is an object of the invention to provide solid oral dosage forms comprising ezetimibe and pitavastatin at different dose strengths (e.g. 2/10 mg, 4/10 mg) without requiring significant modification of the compositions, i.e. of the excipients and their individual weight content. Preparation of the solid oral dosage forms having different dose strengths should be possible from the same preparation(s) as starting materials merely by relatively adjusting the amount thereof.

Still further, it is an object of the invention to provide a solid oral dosage form that can be prepared by a robust and economical manufacturing process with good processibility on laboratory and particularly on industrial scale, without the need for use of specific technological equipment or to use several technological steps.

The invention aims at developing and preparing a solid dosage form comprising ezetimibe and pitavastatin that has a similar or even faster dissolution profile compared to Ezetrol^{®} and Livalo^{®} and that successfully overcomes the drawbacks of the prior art.

One or more of the above objects have been achieved by the subject-matter of the patent claims.

Figure 1 shows a dissolution profile of ezetimibe for Examples 1-4 in basic media.

Figure 2 shows a dissolution profile of ezetimibe for Examples 1-4 in acidic media.

Figure 3 shows a dissolution profile of pitavastatin for Examples 1-4 in acidic media.

A first aspect of the invention relates to a solid pharmaceutical composition comprising or essentially consisting of
- an intragranular phase comprising or essentially consisting of
   (i) ezetimibe, a pharmaceutically acceptable salt and/or solvate thereof;
   (ii) one or more intragranular diluents;
   (iii) one or more intragranular binders;
   (iv) optionally, one or more intragranular disintegrants; and
   (v) optionally, one or more intragranular surfactants; and
- an extragranular phase comprising or essentially consisting of
   (vi) pitavastatin, a pharmaceutically acceptable salt and/or solvate thereof;
   (vii) one or more extragranular diluents;
   (viii) optionally, one or more extragranular disintegrants; and
   (ix) optionally, one or more extragranular lubricants.

In preferred embodiments, the one or more intragranular diluents are selected from monosaccharides, disaccharides, oligosaccharides and sugar alcohols; preferably lactose or mannitol; and/or the one or more intragranular binders are selected from polyvinylpyrrolidone.

In particularly preferred embodiments, the solid pharmaceutical composition according to the invention form comprises or essentially consists of
- an intragranular phase comprising or essentially consisting of
   (i) ezetimibe, a pharmaceutically acceptable salt and/or solvate thereof;
   (ii) one or more intragranular diluents selected from monosaccharides, disaccharides, oligosaccharides, and sugar alcohols;
   (iii) one or more intragranular binders selected from polyvinylpyrrolidone;
   (iv) optionally, one or more intragranular disintegrants; and
   (v) optionally, one or more intragranular surfactants; and
- an extragranular phase comprising or essentially consisting of
   (vi) pitavastatin, a pharmaceutically acceptable salt and/or solvate thereof;
   (vii) one or more extragranular diluents;
   (viii) optionally, one or more extragranular disintegrants; and
   (ix) optionally, one or more extragranular lubricants;
   wherein the extragranular phase does not contain a binder selected from cellulose ethers; preferably not any binder.

The invention also relates to oral dosage forms comprising or essentially consisting of the pharmaceutical composition according to the invention.

The oral dosage form according to the invention is a solid dosage form comprising a common blend (pharmaceutical composition) of the intragranular phase and the extragranular phase. Preferably, the oral dosage form is a tablet, coated or uncoated, or a capsule. Preferably, the oral dosage form according to the invention is a single layer tablet, optionally a coated single layer tablet; preferably an uncoated single layer tablet.

It has been surprisingly found that the oral dosage forms according to the invention show at least a similar dissolution profile compared to that of reference products Ezetrol^{®}, and Livalo^{®}.

Further, it has been surprisingly found that the solid pharmaceutical compositions and the oral dosage forms according to the invention can be manufactured by conventional and economical manufacturing processes. In particular, a common mixture can be prepared in a single vessel without the need for two separate granulation processes. Further, oral dosage forms can be provided in different dose strengths (e.g. 2/10 mg, 4/10 mg,) without requiring significant modification of the formulation, i.e. the formulation of the ezetimibe granulate remains the same and only the extra-granular pitavastatin formulation needs to be varied.

Still further, it has been surprisingly found that oral solid dosage forms according to the invention provide fast disintegration and quick release of ezetimibe. This is in contrast to oral dosage forms known from the prior art, comprising two layers or two separate granulates of each active ingredient, which as shown by way of comparative example, show slower dissolution profiles.

According to the invention and unless specified otherwise, all amount indications are provided on a weight basis or weight/weight basis, as appropriate. If the active pharmaceutical substance is used in the form of a pharmaceutically acceptable salt, the weight of the entire salt is to be considered, including the weight component of the counter ion. If the active pharmaceutical substance is used in the form of a solvate or hydrate, the additional weight associated with solvent or water components in the substance is to be disregarded. That is, a theoretical weight of the anhydrous pure substance (or its pharmaceutically acceptable salt and/or hydrate or solvate, if appropriate) is to be calculated and considered in connection with the invention.

The invention pertains to a solid pharmaceutical composition and to an oral dosage form comprising ezetimibe and pitavastatin, wherein ezetimibe and pitavastatin are physically separated from each other but present in a common blend, which provides an improved dissolution profile, excellent physical stability, improved chemical stability, good bioavailability and long shelf-life. The solid pharmaceutical composition and the oral dosage form according to the invention can be prepared by a robust and economical manufacturing process with good processibility on laboratory and industrial level.

The solid pharmaceutical compositions and oral dosage forms according to the invention comprise ezetimibe including its salts, solvates, hydrates, enantiomers and any polymorphs thereof in a combination with pitavastatin including its salts, solvates, hydrates, enantiomers and any polymorphs thereof, wherein ezetimibe and pitavastatin are physically separated from each other but present in the common blend. The common blend (pharmaceutical composition) can be further compressed into a tablet such as a single layer tablet, or filled into a capsule.

The expression *"physically separated from each other"* as used herein designates that at least two active substances are not in intimate contact and that they are separated from each other (except at the surface of the granule). The active ingredients are not intimately mixed with each other, and the contact between them is reduced.

The term *"common blend"* as used herein designates the mixture of the ezetimibe intragranular phase and the pitavastatin extragranular phase, which both contain one or more pharmaceutically acceptable excipients. The terms "common blend" and the "pharmaceutical composition" can be used interchangeably.

The terms *"intragranular phase"* and *"extragranular phase"* are known to the skilled person and are used in the conventional meaning. A common blend and pharmaceutical composition, respectively, contains a granulate comprising ezetimibe and one or more excipients, which are mixed with pitavastatin and one or more excipients. The granulate and its constituents forms the intragranular phase, whereas the pitavastatin and one or more excipients form the extragranular phase.

The solid pharmaceutical composition and oral dosage form according to the invention comprise ezetimibe and pitavastatin as the active substances in a common blend and one or more pharmaceutically acceptable excipients such as one or more diluents, one or more lubricants, one or more glidants, one or more disintegrants, one or more binders, one or more surfactants, one or more stabilizing agents, and the like.

The term *"excipient"* as used herein refers to any pharmaceutically acceptable substance that has no therapeutic activity as such. Pharmaceutically acceptable excipients may for example be selected from diluents, binders, disintegrants, surfactants lubricants, and glidants. In preferred embodiments, the solid pharmaceutical composition and oral dosage form according to the invention may further comprise any other pharmaceutically acceptable excipients that can be selected among any known state of the art for solid dosage forms, as described e.g. in Remington: The Science and Practice of Pharmacy, edited by A. Adejare, Academic Press, 23rd edition, 2020; or Pharmaceutical Excipients: Properties, Functionality, and Applications in Research and Industry, edited by O.M.Y. Koo, Wiley, 1st edition 2016.

Individual excipients may have polyfunctional properties in the solid pharmaceutical composition and oral dosage form according to the invention, e.g. may exert both disintegrating and binding properties, or both lubricating and gliding properties, or may exert filling, binding and disintegrating properties.

The intragranular phase of the solid pharmaceutical composition and oral dosage form according to the invention comprises at least one or more intragranular diluents, and one or more intragranular binders. In preferred embodiments, the intragranular phase further comprises one or more intragranular disintegrants and/or one or more intragranular surfactants. The intragranular phase may further comprise one or more pharmaceutically acceptable excipients that may be for example selected from intragranular lubricants, intragranular glidants, intragranular binders, intragranular colorants, and the like.

The extragranular phase of the solid pharmaceutical composition and oral dosage form according to the invention comprises at least one or more extragranular diluents. In preferred embodiments, the extragranular phase further comprises one or more extragranular disintegrants and/or one or more extragranular lubricants. The extragranular phase may further comprise one or more pharmaceutically acceptable excipients that may be for example selected from extragranular glidants, extragranular stabilizing agents (e.g. basic alkaline or alkaline earth metal salts), extragranular surfactants, extragranular colorants, and the like.

The intragranular phase of the pharmaceutical composition according to the invention comprises ezetimibe, a pharmaceutically acceptable salt and/or solvate thereof.

The term *"ezetimibe"* as used herein designates ezetimibe (CAS 163222-33-1; ATC C10AX09) in its any known form or any polymorph form known from the state of the art. Ezetimibe used in the solid pharmaceutical composition and oral dosage form according to the invention may be prepared according to any manufacturing process known from the state of the art.

Ezetimibe in accordance with the invention is preferably characterized by particle size distribution (PSD) as determined by the laser diffraction method such as Malvem Master Sizer, preferably in accordance with USP <429> *"Laser Diffraction Measurement of Particle Size".*

In preferred embodiments, ezetimibe particles have a d(0.9) value of less than 50 µm, preferably less than 30 µm, and more preferably less than 15 µm. In preferred embodiments, ezetimibe particles have a d(0.5) value of less than 20 µm, preferably less than 15 µm, and more preferably less than 10 µm. In preferred embodiments, ezetimibe particles have a d(0.1) value of less than 15 µm, preferably less than 10 µm, and more preferably less than 5 µm.

Preferably, the ezetimibe, the pharmaceutically acceptable salt and/or solvate thereof is present in non-salt form.

In preferred embodiments of the invention, the ezetimibe, pharmaceutically acceptable salt and/or solvate thereof is present in the non-salt non-solvate form of ezetimibe.

Preferably, the ezetimibe, the pharmaceutically acceptable salt and/or solvate thereof is present in crystalline form.

Preferably, essentially the total amount of the ezetimibe, the pharmaceutically acceptable salt and/or solvate thereof that is contained in the pharmaceutical composition is present in the intragranular phase.

In preferred embodiments, ezetimibe is present in an amount from 0.1 to 12 wt.-%, preferably 0.5 to 8.0 wt.-%, and more preferably 1.0 to 6.0 wt.-% of the pharmaceutical composition.

Preferably, the content of the ezetimibe, the pharmaceutically acceptable salt and/or solvate thereof is within the range of 3.5±3.3 wt.-%, preferably 3.5±3.0 wt.-%, more preferably 3.5±2.7 wt.-%, still more preferably 3.5±2.4 wt.-%, yet more preferably 3.5±2.1 wt.-%, even more preferably 3.5±1.8 wt.-%, most preferably 3.5±1.5 wt.-%, and in particular 3.5±1.2 wt.-%, in each case relative to the total weight of the pharmaceutical composition.

The extragranular phase of the pharmaceutical composition according to the invention comprises pitavastatin, a pharmaceutically acceptable salt and/or solvate thereof.

The term *"pitavastatin"* as used herein designates pitavastatin (CAS 147511-69-1; ATC C10AA08) in any of its pharmaceutically acceptable salts, solvates, hydrates, enantiomers and any mixture thereof in any pharmaceutically acceptable polymorphic form. Preferably, pitavastatin is present in the form of pitavastatin hemi calcium (2:1) salt (CAS 147526-32-7). Pitavastatin used in the solid pharmaceutical composition and oral dosage form according to the invention may be prepared according to any manufacturing process known from the state of the art.

Pitavastatin in accordance with the invention is preferably characterized by particle size distribution (PSD) as determined by the laser diffraction method such as Malvern Master Sizer, preferably in accordance with USP <429> *"Laser Diffraction Measurement of Particle Size".*

In preferred embodiments, pitavastatin particles have a d(0.9) value of less than 70 µm, preferably less than 50 µm, and more preferably less than 30 µm. In preferred embodiments, pitavastatin particles have a d(0.5) value of less than 40 µm, preferably less than 25 µm, and more preferably less than 15 µm. In preferred embodiments, pitavastatin particles have a d(0.1) value of less than 15 µm, preferably less than 10 µm, and more preferably less than 5 µm.

Preferably, the pitavastatin, the pharmaceutically acceptable salt and/or solvate thereof is present in form of its hemi calcium salt.

Preferably, the pitavastatin, the pharmaceutically acceptable salt and/or solvate thereof is present in crystalline form.

Preferably, essentially the total amount of the pitavastatin, the pharmaceutically acceptable salt and/or solvate thereof that is contained in the pharmaceutical composition is present in the extragranular phase.

In preferred embodiments, pitavastatin is present in the amount from 0.1 to 10 wt.-%, preferably 0.2 to 8.0 wt.-%, and more preferably 0.5 to 5.0 wt.-% of the pharmaceutical composition.

Preferably, the content of the pitavastatin, the pharmaceutically acceptable salt and/or solvate thereof is within the range of 1.2±1.1 wt.-%, preferably 1.2±1.0 wt.-%, more preferably 1.2±0-9 wt.-%, still more preferably 1.2±0.8 wt.-%, yet more preferably 1.2±0.7 wt.-%, even more preferably 1.2±0.6 wt.-%, most preferably 1.2±0.5 wt.-%, and in particular 1.2±0.4 wt.-%, in each case relative to the total weight of the pharmaceutical composition.

The solid pharmaceutical composition and oral dosage form according to the invention comprise diluents (fillers).

Diluents are preferably selected from, but not limited to
- lactose (e.g. anhydrous or hydrate or amorphous (partially or completely));
- polysaccharides, (e.g. starches or celluloses); starches may be selected from partially or wholly pregelatinized starch, com starch, wheat starch, rice starch, tapioca starch, potato starch and any mixture thereof; celluloses may be selected from powdered cellulose, microcrystalline cellulose, silicified microcrystalline cellulose, microcrystalline cellulose co-processed with other excipients such as lactose, starch, silicon dioxide, mannitol, etc. and any mixture thereof;
- monosaccharides (e.g. glucose, fructose);
- disaccharides (e.g. sucrose, lactose monohydrate, anhydrous lactose, α-lactose, β-lactose, isomaltose, trehalose);
- oligosaccharides (e.g. raffinose, dextrates);
- compressible sugars;
- sugar alcohols (e.g. mannitol, erythritol, sorbitol, maltitol, xylitol, lactitol);
- inorganic salts of phosphoric acid;
- inorganic salts.

Mannitol, microcrystalline cellulose that may optionally be silicified, and lactose are preferred diluents.

The intragranular phase of the pharmaceutical composition according to the invention comprises one or more intragranular diluents.

Preferably, the one or more intragranular diluents are independently selected from monosaccharides, disaccharides, oligosaccharides, and sugar alcohols. Preferably, the one or more intragranular diluents are independently selected from the group consisting of lactose, lactose monohydrate, anhydrous lactose, α-lactose, glucose, sucrose, mannitol, sorbitol, maltitol, xylitol, lactitol, and erythritol, and mixtures thereof; more preferably lactose or mannitol. Preferably, the one or more intragranular diluents are selected from crystalline lactose and mannitol.

Preferably, the intragranular phase comprises a single intragranular diluent.

Preferably, the one or more intragranular diluents comprise or essentially consist of lactose; preferably crystalline lactose.

Preferably, the one or more intragranular diluents comprise or essentially consist of mannitol.

Preferably, the one or more intragranular diluents differ from the extragranular diluents.

Preferably, the total content of the one or more intragranular diluents is at least 2.0 wt.-%, preferably at least 4.0 wt.-%, more preferably at least 6.0 wt.-%, still more preferably at least 8.0 wt.-%, yet more preferably at least 10 wt.-%, even more preferably at least 12 wt.-%, most preferably at least 14 wt.-%, and in particular at least 16 wt.-%, in each case relative to the total weight of the pharmaceutical composition.

Preferably, the total content of the one or more intragranular diluents is within the range of 25±23 wt.-%, preferably 25±21 wt.-%, more preferably 25±19 wt.-%, still more preferably 25±17 wt.-%, yet more preferably 25±15 wt.-%, even more preferably 25±13 wt.-%, most preferably 25±11 wt.-%, and in particular 25±9 wt.-%, in each case relative to the total weight of the pharmaceutical composition.

Preferably, the total content of the one or more intragranular diluents; preferably lactose; more preferably crystalline lactose; is within the range of 18±16 wt.-%, preferably 18±14 wt.-%, more preferably 18±12 wt.-%, still more preferably 18±10 wt.-%, yet more preferably 18±8 wt.-%, even more preferably 18±6 wt.-%, most preferably 18±4 wt.-%, and in particular 18±2 wt.-%, in each case relative to the total weight of the pharmaceutical composition.

The extragranular phase of the pharmaceutical composition according to the invention comprises one or more extragranular diluents.

In preferred embodiments, the one or more extragranular diluents are independently selected from the group consisting of
- lactose; preferably anhydrous lactose, lactose hydrate, partially amorphous lactose, completely amorphous lactose, which can be milled, sieved, micronized, granulated/agglomerated, or spray dried;
- starch or starch derivatives; preferably partially or wholly pregelatinized starch, com starch, wheat starch, rice starch, tapioca starch, potato starch;
- cellulose or cellulose derivatives; preferably powdered cellulose, microcrystalline cellulose, silicified microcrystalline cellulose, co-processed microcrystalline cellulose with other excipients such as lactose, starch, silicon dioxide, and mannitol;
- monosaccharides, disaccharides, oligosaccharides and sugar alcohols; preferably glucose, fructose, sucrose, lactose monohydrate, anhydrous lactose, α-lactose, β-lactose, raffinose, isomaltose, trehalose, dextrates, mannitol, erythritol, sorbitol, maltitol, xylitol, lactitol, or compressible sugars; and mixture thereof; preferably microcrystalline cellulose which may optionally be silicified, lactose monohydrate, and mixtures thereof; more preferably microcrystalline cellulose which may optionally be silicified.

Preferably, the one or more extragranular diluents are independently selected from monosaccharides, disaccharides, oligosaccharides, and sugar alcohols; preferably from lactose and cellulose; more preferably from lactose monohydrate, crystalline lactose, and microcrystalline cellulose.

Preferably, the extragranular phase comprises two or three extragranular diluents; preferably two extragranular diluents.

Preferably, the one or more extragranular diluents comprise or essentially consist of lactose and microcrystalline cellulose; more preferably lactose monohydrate and microcrystalline cellulose; or lactose monohydrate, crystalline lactose and microcrystalline cellulose.

According to a particularly preferred embodiment, the one or more extragranular diluents are selected from cellulose powder, microcrystalline cellulose and/or lactose anhydrous, lactose hydrate, modified lactose such as agglomerated lactose. More preferably, the extragranular phase comprises at least two diluents, preferably selected from cellulose and lactose.

Preferably, microcrystalline cellulose has an average particle size from 10 µm to 200 µm, preferably from 20 to 150 µm, and a moisture content ≤ 5%. Preferably, microcrystalline cellulose has (i) an average particle size of 20 µm and a moisture content ≤ 5%, such as Avicel PH-105; (ii) an average particle size of 50 µm and a moisture content ≤ 5%, such as Avicel PH-101 or Vivapur 101; (iii) an average particle size of 50 µm and a moisture content ≤ 2%, such as Avicel PH-1 13; (iv) an average particle size of from 90 to 120 µm and a moisture content ≤ 5%, such as Avicel PH-102 or Vivapur 102; or (v) an average particle size of from 90 to 120 µm and a moisture content ≤ 1.5%, such as Avicel PH-1 12.

It has been surprisingly found that a mixture of microcrystalline cellulose and lactose makes the compression process applicable for the manufacture of the pharmaceutical composition showing desired chemical stability, dose uniformity, weight variation and flow properties.

Preferably, lactose is selected from lactose hydrate with average particle size from 50 µm to 300 µm, preferably from 100 µm to 200 µm. Preferably, lactose is selected from agglomerated lactose such as Tablettose 80, or milled lactose hydrate with average particle size about 5 - 300 µm such as lactose 80 to 200 mesh. Preferably, a combination of two different types of lactose is used.

The weight ratio between lactose, preferably agglomerated lactose to crystalline cellulose, preferably milled crystalline cellulose, is preferably within the range of from 10:1 to 1:1.

The amount of extragranular microcrystalline cellulose is preferably in the range of about 3.0 to about 60 wt.-%, relative to the weight of the extragranular phase. Preferably, the amount of extragranular microcrystalline cellulose is from about 10 to about 50 wt.-%, more preferably from about 30 to about 45 wt.-%, relative to the weight of the extragranular phase.

The amount of extragranular lactose, preferably a mixture of extragranular agglomerated lactose with extragranular milled crystalline lactose, is preferably in the range of about 10 to about 70 wt.-%, relative to the weight of the extragranular phase. Preferably, the amount of lactose is about 30 to about 60 wt.-%, more preferably about 40 to about 50 wt.-%, relative to the weight of the extragranular phase.

Preferably, the total content of the one or more extragranular diluents is at least 15 wt.-%, preferably at least 20 wt.-%, more preferably at least 25 wt.-%, still more preferably at least 30 wt.-%, yet more preferably at least 35 wt.-%, even more preferably at least 40 wt.-%, most preferably at least 45 wt.-%, and in particular at least 50 wt.-%, in each case relative to the total weight of the pharmaceutical composition.

Preferably, the total content of the one or more intragranular diluents is within the range of 60±35 wt.-%, preferably 60±30 wt.-%, more preferably 60±25 wt.-%, still more preferably 60±20 wt.-%, yet more preferably 60±15 wt.-%, even more preferably 60±10 wt.-%, most preferably 60±8 wt.-%, and in particular 60±9 wt.-%, in each case relative to the total weight of the pharmaceutical composition.

Preferably, the one or more extragranular diluents comprise lactose; preferably lactose monohydrate and/or crystalline lactose; and wherein the total content of the lactose is within the range of 39±22.5 wt.-%, preferably 39±20 wt.-%, more preferably 39±17.5 wt.-%, still more preferably 39±15 wt.-%, yet more preferably 39±12.5 wt.-%, even more preferably 39±10 wt.-%, most preferably 39±7.5 wt.-%, and in particular 39±5.0 wt.-%, in each case relative to the total weight of the pharmaceutical composition.

Preferably, the one or more extragranular diluents comprise cellulose; preferably microcrystalline cellulose; and wherein the total content of the cellulose is within the range of 25±22.5 wt.-%, preferably 25±20 wt.-%, more preferably 25±17.5 wt.-%, still more preferably 25±15 wt.-%, yet more preferably 25±12.5 wt.-%, even more preferably 25±10 wt.-%, most preferably 25±7.5 wt.-%, and in particular 25±5.0 wt.-%, in each case relative to the total weight of the pharmaceutical composition.

Preferably, the overall content of the one or more intragranular diluents and the one or more extragranular diluents is at least 50 wt.-%, preferably at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%, in each case relative to the total weight of the pharmaceutical composition.

Preferably, the overall content of the one or more intragranular diluents and the one or more extragranular diluents is within the range of 85±10 wt.-%, preferably 85±9.0 wt.-%, more preferably 85±8.0 wt.-%, still more preferably 85±7.0 wt.-%, yet more preferably 85±6.0 wt.-%, even more preferably 85±5.0 wt.-%, most preferably 85±4.0 wt.-%, and in particular 85±3.0 wt.-%, in each case relative to the total weight of the pharmaceutical composition.

The solid pharmaceutical composition and oral dosage form according to the invention comprise at least one binder, namely one or more intragranular binders.

Binders are preferably selected from, but not limited to
- povidone (polyvinylpyrrolidone);
- copovidone (vinylpyrrolidone- vinyl acetate copolymer);
- cellulose derivatives such as cellulose esters or cellulose ethers (e.g. hydroxymethyl cellulose (HMC), hydroxyethyl cellulose (HEC), hydroxypropyl cellulose (HPC), low substituted hydroxypropyl cellulose and hydroxypropyl methyl cellulose (HPMC)):
- polyvinyl alcohol,
- starch (e.g. corn starch, potato starch, or rice starch); α-starch, pregelatinized starch;
- dextrins;
- gum arabic;
- pullulan;
- poly(meth)acrylates.

The intragranular phase of the pharmaceutical composition according to the invention comprises one or more intragranular binders.

Preferably, the one or more intragranular binders are independently selected from povidone, copovidone, cellulose esters, cellulose ethers, polyvinyl alcohol, starch, α-starch, pregelatinized starch, dextrin, gum arabic, pullulan, and poly(meth)acrylates.

Preferably, the intragranular phase comprises a single intragranular binder.

Preferably, the intragranular phase comprises a polyvinylpyrrolidone or copovidone (vinylpyrrolidone-vinyl acetate copolymer), preferably polyvinylpyrrolidone.

Preferably, the one or more intragranular binders comprise or essentially consist of polyvinylpyrrolidone.

Preferably, the total content of the one or more intragranular binders is within the range of 1.5±1.2 wt.-%, preferably 1.5±1.1 wt.-%, more preferably 1.5±1.0 wt.-%, still more preferably 1.5±0.9 wt.-%, yet more preferably 1.5±0.8 wt.-%, even more preferably 1.5±0.7 wt.-%, most preferably 1.5±0.6 wt.-%, and in particular 1.5±0.5 wt.-%, in each case relative to the total weight of the pharmaceutical composition.

Preferably, the extragranular phase does not contain any extragranular binder selected from cellulose ethers; preferably not any extragranular binder. In preferred embodiments, the extragranular phase is free of binders selected form povidone (polyvinylpyrrolidone), copovidone (vinylpyrrolidone-vinyl acetate copolymer), cellulose derivatives such as cellulose esters or cellulose ethers (e.g. hydroxymethyl cellulose, hydroxyethyl cellulose, hydroxypropyl cellulose, low substituted hydroxypropyl cellulose and hydroxypropyl methyl cellulose), polyvinyl alcohol.

The solid pharmaceutical composition and oral dosage form according to the invention may comprise one or more disintegrants.

Disintegrants are preferably selected from, but not limited to
- crospovidone;
- starch, maize starch, pregelatinized starch, sodium starch glycollate, modified starch, hydroxypropyl starch, carboxymethyl starch;
- sodium and/or calcium salts of carboxymethyl cellulose, cross-linked carboxymethylcellulose (e.g. croscarmellose sodium and/or croscarmellose calcium);
- polacrilin potassium;
- alginic acid or alginates, sodium and/or calcium alginate;
- polyacrylates;
- docusate sodium;
- methylcellulose; and
- agar, gums, guar gum, chitosan.

Preferred disintegrants are croscarmellose sodium, crospovidone and mixtures thereof.

Optionally and preferably, the intragranular phase of the pharmaceutical composition according to the invention comprises one or more intragranular disintegrants.

Preferably, the one or more intragranular disintegrants are independently selected from crospovidone, starch, pregelatinized starch, sodium starch glycollate, modified starch, hydroxypropyl starch, carboxymethyl starch, carboxymethyl cellulose, sodium carboxymethyl cellulose, calcium carboxymethyl cellulose, cross-linked carboxymethylcellulose, sodium croscarmellose, calcium croscarmellose, polacrilin potassium, alginic acid, alginates, sodium alginate, calcium alginate, polyacrylates, docusate sodium, methylcellulose, agarchitosan, gums, guar gum, and mixtures thereof; preferably from sodium croscarmellose and crospovidone; more preferably sodium croscarmellose.

Preferably, the intragranular phase comprises a single intragranular disintegrant.

Preferably, the one or more intragranular disintegrants comprise or essentially consist of sodium croscarmellose.

Preferably, the one or more intragranular disintegrants differ from the one or more extragranular disintegrants.

Preferably, the one or more intragranular disintegrants are identical to the one or more extra-granular disintegrants. In preferred embodiments, the intragranular phase and the extragranular phase comprise the same disintegrant, preferably sodium croscarmellose.

Preferably, the total content of the one or more intragranular disintegrants is within the range of 1.5±1.2 wt.-%, preferably 1.5±1.1 wt.-%, more preferably 1.5±1.0 wt.-%, still more preferably 1.5±0.9 wt.-%, yet more preferably 1.5±0.8 wt.-%, even more preferably 1.5±0.7 wt.-%, most preferably 1.5±0.6 wt.-%, and in particular 1.5±0.5 wt.-%, in each case relative to the total weight of the pharmaceutical composition.

Optionally an preferably, the extragranular phase of the pharmaceutical composition according to the invention comprises one or more extragranular disintegrants.

Preferably, the one or more extragranular disintegrants are independently selected from crospovidone, starch, pregelatinized starch, sodium starch glycollate, modified starch, hydroxypropyl starch, carboxymethyl starch, carboxymethyl cellulose, sodium carboxymethyl cellulose, calcium carboxymethyl cellulose, cross-linked carboxymethylcellulose, sodium croscarmellose, calcium croscarmellose, polacrilin potassium, alginic acid, alginates, sodium alginate, calcium alginate, polyacrylates, docusate sodium, methylcellulose, agarchitosan, gums, guar gum, and mixtures thereof; preferably from sodium croscarmellose and crospovidone; more preferably sodium croscarmellose.

Preferably, the extragranular phase comprises a single extragranular disintegrant.

Preferably, the one or more extragranular disintegrants comprise or essentially consist of sodium croscarmellose.

Preferably, the total content of the one or more extragranular disintegrants is within the range of 5.5±4.0 wt.-%, preferably 5.5±3.5 wt.-%, more preferably 5.5±3.0 wt.-%, still more preferably 5.5±2.5 wt.-%, yet more preferably 5.5±2.0 wt.-%, even more preferably 5.5±1.5 wt.-%, most preferably 5.5±1.0 wt.-%, and in particular 5.5±0.5 wt.-%, in each case relative to the total weight of the pharmaceutical composition.

Preferably, the overall content of the one or more intragranular disintegrants and the one or more extragranular disintegrants is at least 2.0 wt.-%, preferably at least 2.5 wt.-%, more preferably at least 3.0 wt.-%, still more preferably at least 3.5 wt.-%, yet more preferably at least 4.0 wt.-%, even more preferably at least 4.5 wt.-%, most preferably at least 5.5 wt.-%, and in particular at least 6.0 wt.-%, in each case relative to the total weight of the pharmaceutical composition.

Preferably, the overall content of the one or more intragranular disintegrants and the one or more extragranular disintegrants is within the range of 6.9±2.4 wt.-%, preferably 6.9±2.1 wt.-%, more preferably 6.9±1.8 wt.-%, still more preferably 6.9±1.5 wt.-%, yet more preferably 6.9±1.2 wt.-%, even more preferably 6.9±0.9 wt.-%, most preferably 6.9±0.6 wt.-%, and in particular 6.9±0.3 wt.-%, in each case relative to the total weight of the pharmaceutical composition.

The solid pharmaceutical composition and oral dosage form according to the invention may comprise one or more surfactants (wetting agents).

Surfactants are preferably selected from, but not limited to,
- cationic surfactants;
- anionic surfactants; such as carboxylates: alkyl carboxylates-fatty acid salts; carboxylate fluoro surfactants; sulfates: alkyl sulfates (e.g., sodium lauryl sulfate); alkyl ether sulfates (e.g., sodium laureth sulfate); sulfonates: docusates (e.g., dioctyl sodium sulfosuccinate); alkyl benzene sulfonates; phosphate esters: alkyl aryl ether phosphates; alkyl ether phosphates;
- zwitterionic surfactants; and
- non-ionic surfactants; such as polyol esters {e.g. glycol esters, glycerol esters, and sorbitan derivatives [such as fatty acid esters of sorbitan (generally referred to as Spans) and their ethoxylated derivatives (generally referred to as Tweens)]}, polyoxyethylene esters, poloxamers.

Preferred surfactants are polysorbate 80 and sodium lauryl sulfate.

Optionally and preferably, the intragranular phase of the pharmaceutical composition according to the invention comprises one or more intragranular surfactants.

Preferably, the one or more intragranular surfactants are independently selected from cationic surfactants, anionic surfactants, zwitterionic surfactants and non-ionic surfactants; preferably anionic surfactants; more preferably anionic surfactants selected from carboxylates, alkyl carboxylates-fatty acid salts, carboxylate fluoro surfactants, sulfates, alkyl sulfates, alkyl ether sulfates, sulfonates, docusates, alkyl benzene sulfonates, phosphate esters, alkyl aryl ether phosphates, and alkyl ether phosphates; still more preferably alkyl sulfates; yet more preferably sodium lauryl sulfate.

Preferably, the intragranular phase comprises a single intragranular surfactant.

Preferably, the one or more intragranular surfactants comprise or essentially consist of sodium lauryl sulfate or polysorbate 80.

Preferably, the total content of the one or more intragranular surfactants is within the range of 1.5±1.4 wt.-%, preferably 1.5±1.3 wt.-%, more preferably 1.5±1.2 wt.-%, still more preferably 1.5±1.1 wt.-%, yet more preferably 1.5±1.0 wt.-%, even more preferably 1.5±0.9 wt.-%, most preferably 1.5±0.8 wt.-%, and in particular 1.5±0.7 wt.-%, in each case relative to the total weight of the pharmaceutical composition.

Preferably, the extragranular phase does not contain any extragranular surfactant.

Optionally an preferably, the extragranular phase of the pharmaceutical composition according to the invention comprises one or more extragranular lubricants.

Lubricants are preferably selected from, but not limited to
- fatty acids (i.e. carboxylic acids with 12 to 20 carbon atoms);
- fatty acid esters including glyceride esters such as glyceryl monostearate, glyceryl tribehenate, or glyceryl dibehenate (e.g. Compritol^{®} 888);
- metal salts of fatty acids, including magnesium, calcium, aluminum or zinc salts of fatty acids (e.g. magnesium, calcium, aluminum or zinc stearate, magnesium palmitate, or magnesium oleate);
- hydrogenated vegetable oil, hydrogenated castor oil;
- waxes (e.g. Sterotex^{®} NL, Lubriwax^{®} [hydrogenated vegetable oil type], meads wax or spermaceti);
- boric acid;
- sodium stearyl fumarate;
- polymers (e.g., PEG, macrogols);
- sugar esters such as sorbitan monostearate and sucrose monopalmitate.

Preferred lubricants are magnesium stearate and sodium stearyl fumarate.

Preferably, the one or more extragranular lubricants are independently selected from fatty acids, fatty acid esters, fatty acid glyceride esters, metal salts of fatty acids, hydrogenated vegetable oil, hydrogenated castor oil, waxes, boric acid, sodium stearyl fumarate, macrogols, and sugar esters; preferably from magnesium stearate and sodium stearyl fumarate; more preferably magnesium stearate.

Preferably, the extragranular phase comprises a singly extragranular lubricant.

Preferably, the one or more extragranular lubricants comprise or essentially consist of magnesium stearate.

Preferably, the total content of the one or more extragranular lubricants is within the range of 0.9±0.8 wt.-%, preferably 0.9±0.7 wt.-%, more preferably 0.9±0.6 wt.-%, still more preferably 0.9±0.5 wt.-%, yet more preferably 0.9±0.4 wt.-%, even more preferably 0.9±0.3 wt.-%, most preferably 0.9±0.2 wt.-%, and in particular 0.9±0.1 wt.-%, in each case relative to the total weight of the pharmaceutical composition.

Preferably, the intragranular phase does not contain any intragranular lubricant.

Preferably, the intragranular phase is wet granulated; preferably with a aqueous solvent; more preferably water.

Preferably, the total content of the intragranular phase is at least 7.5 wt.-%, preferably at least 10 wt.-%, more preferably at least 12.5 wt.-%, still more preferably at least 15 wt.-%, yet more preferably at least 17.5 wt.-%, even more preferably at least 20 wt.-%, most preferably at least 22.5 wt.-%, and in particular at least 6.0 wt.-%, in each case relative to the total weight of the pharmaceutical composition.

Preferably, the total content of the intragranular phase is within the range of 32±16 wt.-%, preferably 32±15 wt.-%, more preferably 32±14 wt.-%, still more preferably 32±13 wt.-%, yet more preferably 32±12 wt.-%, even more preferably 32±11 wt.-%, most preferably 32±10 wt.-%, and in particular 32±9.0 wt.-%, in each case relative to the total weight of the pharmaceutical composition.

Preferably, the total content of the extragranular phase is at least 40 wt.-%, preferably at least 42.5 wt.-%, more preferably at least 45 wt.-%, still more preferably at least 47.5 wt.-%, yet more preferably at least 50 wt.-%, even more preferably at least 52.5 wt.-%, most preferably at least 55 wt.-%, and in particular at least 57.5 wt.-%, in each case relative to the total weight of the pharmaceutical composition.

Preferably, the total content of the extragranular phase is within the range of 68±16 wt.-%, preferably 68±15 wt.-%, more preferably 68±14 wt.-%, still more preferably 68±13 wt.-%, yet more preferably 68±12 wt.-%, even more preferably 68±11 wt.-%, most preferably 68±10 wt.-%, and in particular 68±9.0 wt.-%, in each case relative to the total weight of the pharmaceutical composition.

Preferably, besides the ezetimibe, the pharmaceutically acceptable salt and/or solvate thereof and the pitavastatin, the pharmaceutically acceptable salt and/or solvate thereof does not contain any additional pharmacologically active ingredient.

Another aspect of the invention relates to an oral dosage form comprising or essentially consisting of the pharmaceutical composition according to the invention as described above.

In preferred embodiments, the dosage form is a tablet; preferably a single layer tablet. Preferably, the tablet is not coated.

In preferred embodiments, the tablet in an uncoated tablet. By the pharmaceutical composition of the present invention, the desired chemical stability is comparable or even better without the application of the coating layer. Omitting a coating phase provides also for economic and environmentally friendly manufacturing process.

In other preferred embodiments, the tablet is a coated tablet. In case the coating is applied onto the solid pharmaceutical formulation, it is preferably composed of at least one film forming polymer for coating and at least one further pharmaceutically acceptable excipient, which can be selected from, but is not limited to, plasticizers, anti-tacking agents, pigments and coloring agents, pore formers and any mixture thereof. Film forming polymers for coating are preferably selected from, but are not limited to, cellulose ethers such as low molecular weight hydroxypropyl methyl cellulose (HPMC) and low molecular weight hydroxy propyl cellulose (HPC), polyvinyl alcohol, copolymers of vinyl alcohol and ethylene glycol such as Kollicoat^{®} IR and/or Kollicoat^{®} Protect manufactured by BASF. In preferred embodiments, ready to use products such as Opadry^{®} can be used.

The thickness of the coating can be in the range e.g. from 5 to 100 µm, preferably from 10 to 80 µm, and more preferably from 20 to 50 µm.

In other preferred embodiments, the dosage form is a capsule.

Preferably, the dosage form contains
- the ezetimibe, the pharmaceutically acceptable salt and/or solvate thereof at a dosage of 2 mg or 4 mg; expressed as equivalent dose of the non-salt form of ezetimibe; and
- the pitavastatin, the pharmaceutically acceptable salt and/or solvate thereof at a dosage of 10 mg; expressed as equivalent dose of the non-salt form of ezetimibe.

Another aspect of the invention relates to the oral dosage form according to the invention as described above for use in the treatment of hyperlipidemia and/or hypercholesterolemia (primary hypercholesterolemia [heterozygous familial and non-familial], mixed hyperlipidemia or homozygous familial hypercholesterolemia) or for reduction of cardiovascular event risk. Thus, the invention also relates to the use of the pharmaceutical composition according to the invention as described above for the manufacture of an oral dosage form according to the invention as described above for use in the treatment of hyperlipidemia and/or hypercholesterolemia (primary hypercholesterolemia [heterozygous familial and non-familial], mixed hyperlipidemia or homozygous familial hypercholesterolemia) or for reduction of cardiovascular event risk. The invention further relates to a method for the treatment of hyperlipidemia and/or hypercholesterolemia (primary hypercholesterolemia [heterozygous familial and non-familial], mixed hyperlipidemia or homozygous familial hypercholesterolemia) or for reduction of cardiovascular event risk comprising orally administering the pharmaceutical dosage form according to the invention as described above to a subject in need thereof.

Another aspect of the invention relates to a process for the preparation of the pharmaceutical composition according to the invention as described above, the process comprising the steps of
A) preparing an intragranular phase comprising the sub-steps:
   a) dispersing one or more intragranular binders in a suitable liquid; preferably an aqueous liquid or an organic solvent; more preferably water; thereby obtaining a dispersion;
   b) homogenizing ezetimibe, a pharmaceutically acceptable salt and/or solvate thereof in the dispersion obtained in sub-step a) thereby obtaining a suspension;
   c) dispersing one or more intragranular surfactants in the dispersion obtained in step b) thereby obtaining a granulation liquid;
   d) spraying the granulation liquid onto a mixture comprising one or more intragranular diluents, optionally one or more intragranular disintegrants, and optionally one or more further pharmaceutically acceptable intragranular excipients, thereby obtaining granules;
   e) drying and sieving the granules obtained in sub-step d) thereby obtaining the intragranular phase;
B) preparing an extragranular phase comprising the sub steps:
   f) homogenizing pitavastatin, a pharmaceutically acceptable salt and/or solvate thereof optionally together with one or more extragranular pharmaceutically acceptable excipients, preferably by means of a homogenizer, thereby obtaining a homogenous mixture; and
   g) adding one or more extragranular diluents and one or more extragranular disintegrants to the homogenous mixture obtained in sub-step f) thereby obtaining the extragranular phase;
C) preparing a common blend comprising the sub-steps:
   h) blending the intragranular phase obtained in sub-step e) with the extragranular phase obtained in sub-step g);
   i) mixing one or more extragranular lubricants with one or more extragranular diluents to obtain a homogeneous mixture; and
   j) blending the mixtures obtained in sub steps h) and i) to obtain the pharmaceutical composition.

In preferred embodiments, the intragranular phase is prepared by wet granulation; preferably aqueous wet granulation.

In preferred embodiments, the extragranular phase is prepared by mixing pitavastatin and optionally at least pharmaceutically acceptable excipient; preferably no wet or dry granulation is used.

In preferred embodiments, sub-step a) involves dissolving one or more intragranular binders in an organic solvent or in water.

Another aspect of the invention relates to a solid pharmaceutical composition, which is obtainable by the process for the preparation of the pharmaceutical composition according to the invention as described above.

Another aspect of the invention relates to a process for the preparation of an oral dosage form according to the invention as described above, the process comprising the process for the preparation of the pharmaceutical composition according to the invention as described above.

Preferably, the process comprises the additional steps of
D) compressing the pharmaceutical composition prepared in step C) into a tablet; or filling a capsule with the pharmaceutical composition prepared in step C); and
E) optionally, film-coating the tablet obtained in step D).

An other aspect of the invention relates to an oral dosage form, which is obtainable by the process for the preparation of an oral dosage form according to the invention as described above.

Particularly preferred embodiments are summarized as clauses 1 to 72 hereinafter:
Clause 1: A solid pharmaceutical composition comprising or essentially consisting of - an intragranular phase comprising or essentially consisting of (i) ezetimibe, a pharmaceutically acceptable salt and/or solvate thereof; (ii) one or more intragranular diluents; (iii) one or more intragranular binders; (iv) optionally, one or more intragranular disintegrants; and (v) optionally, one or more intragranular surfactants; and - an extragranular phase comprising or essentially consisting of (vi) pitavastatin, a pharmaceutically acceptable salt and/or solvate thereof; (vii) one or more extragranular diluents; (viii) optionally, one or more extragranular disintegrants; and (ix) optionally, one or more extragranular lubricants.
Clause 2: The pharmaceutical composition according to clause 1, wherein the ezetimibe, the pharmaceutically acceptable salt and/or solvate thereof is present in non-salt form.
Clause 3: The pharmaceutical composition according to clause 1 or 2, wherein the ezetimibe, the pharmaceutically acceptable salt and/or solvate thereof is present in crystalline form.
Clause 4: The pharmaceutical composition according to any of the preceding clauses, wherein essentially the total amount of the ezetimibe, the pharmaceutically acceptable salt and/or solvate thereof that is contained in the pharmaceutical composition is present in the intragranular phase.
Clause 5: The pharmaceutical composition according to any of the preceding clauses, wherein the content of the ezetimibe, the pharmaceutically acceptable salt and/or solvate thereof is within the range of 3.5±3.3 wt.-%, preferably 3.5±3.0 wt.-%, more preferably 3.5±2.7 wt.-%, still more preferably 3.5±2.4 wt.-%, yet more preferably 3.5±2.1 wt.-%, even more preferably 3.5±1.8 wt.-%, most preferably 3.5±1.5 wt.-%, and in particular 3.5±1.2 wt.-%, in each case relative to the total weight of the pharmaceutical composition.
Clause 6: The pharmaceutical composition according to any of the preceding clauses, wherein the one or more intragranular diluents are independently selected from monosaccharides, disaccharides, oligosaccharides, and sugar alcohols; preferably from lactose and mannitol; more preferably from crystalline lactose and mannitol.
Clause 7: The pharmaceutical composition according to any of the preceding clauses, wherein the intragranular phase comprises a single intragranular diluent.
Clause 8: The pharmaceutical composition according to any of the preceding clauses, wherein the one or more intragranular diluents comprise or essentially consist of lactose; preferably crystalline lactose.
Clause 9: The pharmaceutical composition according to any of the preceding clauses, wherein the one or more intragranular diluents comprise or essentially consist of mannitol.
Clause 10: The pharmaceutical composition according to any of the preceding clauses, wherein the one or more intragranular diluents differ from the extragranular diluents.
Clause 11: The pharmaceutical composition according to any of the preceding clauses, wherein the total content of the one or more intragranular diluents is at least 2.0 wt.-%, preferably at least 4.0 wt.-%, more preferably at least 6.0 wt.-%, still more preferably at least 8.0 wt.-%, yet more preferably at least 10 wt.-%, even more preferably at least 12 wt.-%, most preferably at least 14 wt.-%, and in particular at least 16 wt.-%, in each case relative to the total weight of the pharmaceutical composition.
Clause 12: The pharmaceutical composition according to any of the preceding clauses, wherein the total content of the one or more intragranular diluents is within the range of 25±23 wt.-%, preferably 25±21 wt.-%, more preferably 25±19 wt.-%, still more preferably 25±17 wt.-%, yet more preferably 25±15 wt.-%, even more preferably 25±13 wt.-%, most preferably 25±11 wt.-%, and in particular 25±9 wt.-%, in each case relative to the total weight of the pharmaceutical composition.
Clause 13: The pharmaceutical composition according to any of the preceding clauses, wherein the total content of the one or more intragranular diluents; preferably lactose; more preferably crystalline lactose; is within the range of 18±16 wt.-%, preferably 18±14 wt.-%, more preferably 18±12 wt.-%, still more preferably 18±10 wt.-%, yet more preferably 18±8 wt.-%, even more preferably 18±6 wt.-%, most preferably 18±4 wt.-%, and in particular 18±2 wt.-%, in each case relative to the total weight of the pharmaceutical composition.
Clause 14: The pharmaceutical composition according to any of the preceding clauses, wherein the one or more intragranular binders are independently selected from povidone, copovidone, cellulose esters, cellulose ethers, polyvinyl alcohol, starch, α-starch, pregelatinized starch, dextrin, gum arabic, pullulan, and poly(meth)acrylates.
Clause 15: The pharmaceutical composition according to any of the preceding clauses, wherein the intragranular phase comprises a single intragranular binder.
Clause 16: The pharmaceutical composition according to any of the preceding clauses, wherein the one or more intragranular binders comprise or essentially consist of polyvinylpyrrolidone.
Clause 17: The pharmaceutical composition according to any of the preceding clauses, wherein the total content of the one or more intragranular binders is within the range of 1.5±1.2 wt.-%, preferably 1.5±1.1 wt.-%, more preferably 1.5±1.0 wt.-%, still more preferably 1.5±0.9 wt.-%, yet more preferably 1.5±0.8 wt.-%, even more preferably 1.5±0.7 wt.-%, most preferably 1.5±0.6 wt.-%, and in particular 1.5±0.5 wt.-%, in each case relative to the total weight of the pharmaceutical composition.
Clause 18: The pharmaceutical composition according to any of the preceding clauses, wherein the extragranular phase does not contain any extragranular binder selected from cellulose ethers; preferably not any extragranular binder.
Clause 19: The pharmaceutical composition according to any of the preceding clauses, wherein the one or more intragranular disintegrants are independently selected from crospovidone, starch, pregelatinized starch, sodium starch glycollate, modified starch, hydroxypropyl starch, carboxymethyl starch, carboxymethyl cellulose, sodium carboxymethyl cellulose, calcium carboxymethyl cellulose, cross-linked carboxymethylcellulose, sodium croscarmellose, calcium croscarmellose, polacrilin potassium, alginic acid, alginates, sodium alginate, calcium alginate, polyacrylates, docusate sodium, methylcellulose, agarchitosan, gums, guar gum, and mixtures thereof; preferably from sodium croscarmellose and crospovidone; more preferably sodium croscarmellose.
Clause 20: The pharmaceutical composition according to any of the preceding clauses, wherein the intragranular phase comprises a single intragranular disintegrant.
Clause 21: The pharmaceutical composition according to any of the preceding clauses, wherein the one or more intragranular disintegrants comprise or essentially consist of sodium croscarmellose.
Clause 22: The pharmaceutical composition according to any of the preceding clauses, wherein the one or more intragranular disintegrants differ from the one or more extragranular disintegrants.
Clause 23: The pharmaceutical composition according to any of the preceding clauses, wherein the one or more intragranular disintegrants are identical to the one or more extragranular disintegrants.
Clause 24: The pharmaceutical composition according to any of the preceding clauses, wherein the total content of the one or more intragranular disintegrants is within the range of 1.5±1.2 wt.-%, preferably 1.5±1.1 wt.-%, more preferably 1.5±1.0 wt.-%, still more preferably 1.5±0.9 wt.-%, yet more preferably 1.5±0.8 wt.-%, even more preferably 1.5±0.7 wt.-%, most preferably 1.5±0.6 wt.-%, and in particular 1.5±0.5 wt.-%, in each case relative to the total weight of the pharmaceutical composition.
Clause 25: The pharmaceutical composition according to any of the preceding clauses, wherein the one or more intragranular surfactants are independently selected from cationic surfactants, anionic surfactants, zwitterionic surfactants and non-ionic surfactants; preferably anionic surfactants; more preferably anionic surfactants selected from carboxylates, alkyl carboxylates-fatty acid salts, carboxylate fluoro surfactants, sulfates, alkyl sulfates, alkyl ether sulfates, sulfonates, docusates, alkyl benzene sulfonates,
phosphate esters, alkyl aryl ether phosphates, and alkyl ether phosphates; still more preferably alkyl sulfates; yet more preferably sodium lauryl sulfate.
Clause 26: The pharmaceutical composition according to any of the preceding clauses, wherein the intragranular phase comprises a single intragranular surfactant.
Clause 27: The pharmaceutical composition according to any of the preceding clauses, wherein the one or more intragranular surfactants comprise or essentially consist of sodium lauryl sulfate or polysorbate 80.
Clause 28: The pharmaceutical composition according to any of the preceding clauses, wherein the total content of the one or more intragranular surfactants is within the range of 1.5±1.4 wt.-%, preferably 1.5±1.3 wt.-%, more preferably 1.5±1.2 wt.-%, still more preferably 1.5±1.1 wt.-%, yet more preferably 1.5±1.0 wt.-%, even more preferably 1.5±0.9 wt.-%, most preferably 1.5±0.8 wt.-%, and in particular 1.5±0.7 wt.-%, in each case relative to the total weight of the pharmaceutical composition.
Clause 29: The pharmaceutical composition according to any of the preceding clauses, wherein the extragranular phase does not contain any extragranular surfactant.
Clause 30: The pharmaceutical composition according to any of the preceding clauses, wherein the pitavastatin, the pharmaceutically acceptable salt and/or solvate thereof is present in form of its hemi calcium salt.
Clause 31: The pharmaceutical composition according to any of the preceding clauses, wherein the pitavastatin, the pharmaceutically acceptable salt and/or solvate thereof is present in crystalline form.
Clause 32: The pharmaceutical composition according to any of the preceding clauses, wherein essentially the total amount of the pitavastatin, the pharmaceutically acceptable salt and/or solvate thereof that is contained in the pharmaceutical composition is present in the extragranular phase.
Clause 33: The pharmaceutical composition according to any of the preceding clauses, wherein the content of the pitavastatin, the pharmaceutically acceptable salt and/or solvate thereof is within the range of 1.2±1.1 wt.-%, preferably 1.2±1.0 wt.-%, more preferably 1.2±0-9 wt.-%, still more preferably 1.2±0.8 wt.-%, yet more preferably 1.2±0.7 wt.-%, even more preferably 1.2±0.6 wt.-%, most preferably 1.2±0.5 wt.-%, and in particular 1.2±0.4 wt.-%, in each case relative to the total weight of the pharmaceutical composition.
Clause 34: The pharmaceutical composition according to any of the preceding clauses, wherein the one or more extragranular diluents are independently selected from monosaccharides, disaccharides, oligosaccharides, and sugar alcohols; preferably from lactose and cellulose; more preferably from lactose monohydrate, crystalline lactose, and microcrystalline cellulose.
Clause 35: The pharmaceutical composition according to any of the preceding clauses, wherein the extragranular phase comprises two or three extragranular diluents; preferably two extragranular diluents.
Clause 36: The pharmaceutical composition according to any of the preceding clauses, wherein the one or more extragranular diluents comprise or essentially consist of lactose and microcrystalline cellulose; more preferably lactose monohydrate and microcrystalline cellulose; or lactose monohydrate, crystalline lactose and microcrystalline cellulose.
Clause 37: The pharmaceutical composition according to any of the preceding clauses, wherein the total content of the one or more extragranular diluents is at least 15 wt.-%, preferably at least 20 wt.-%, more preferably at least 25 wt.-%, still more preferably at least 30 wt.-%, yet more preferably at least 35 wt.-%, even more preferably at least 40 wt.-%, most preferably at least 45 wt.-%, and in particular at least 50 wt.-%, in each case relative to the total weight of the pharmaceutical composition.
Clause 38: The pharmaceutical composition according to any of the preceding clauses, wherein the total content of the one or more intragranular diluents is within the range of 60±35 wt.-%, preferably 60±30 wt.-%, more preferably 60±25 wt.-%, still more preferably 60±20 wt.-%, yet more preferably 60±15 wt.-%, even more preferably 60±10 wt.-%, most preferably 60±8 wt.-%, and in particular 60±9 wt.-%, in each case relative to the total weight of the pharmaceutical composition.
Clause 39: The pharmaceutical composition according to any of the preceding clauses, wherein the one or more extragranular diluents comprise lactose; preferably lactose monohydrate and/or crystalline lactose; and wherein the total content of the lactose is within the range of 39±22.5 wt.-%, preferably 39±20 wt.-%, more preferably 39±17.5 wt.-%, still more preferably 39±15 wt.-%, yet more preferably 39±12.5 wt.-%, even more preferably 39±10 wt.-%, most preferably 39±7.5 wt.-%, and in particular 39±5.0 wt.-%, in each case relative to the total weight of the pharmaceutical composition.
Clause 40: The pharmaceutical composition according to any of the preceding clauses, wherein the one or more extragranular diluents comprise cellulose; preferably microcrystalline cellulose; and wherein the total content of the cellulose is within the range of 25±22.5 wt.-%, preferably 25±20 wt.-%, more preferably 25±17.5 wt.-%, still more preferably 25±15 wt.-%, yet more preferably 25±12.5 wt.-%, even more preferably 25±10 wt.-%, most preferably 25±7.5 wt.-%, and in particular 25±5.0 wt.-%, in each case relative to the total weight of the pharmaceutical composition.
Clause 41: The pharmaceutical composition according to any of the preceding clauses, wherein the one or more extragranular disintegrants are independently selected from crospovidone, starch, pregelatinized starch, sodium starch glycollate, modified starch, hydroxypropyl starch, carboxymethyl starch, carboxymethyl cellulose, sodium carboxymethyl cellulose, calcium carboxymethyl cellulose, cross-linked carboxymethylcellulose, sodium croscarmellose, calcium croscarmellose, polacrilin potassium, alginic acid, alginates, sodium alginate, calcium alginate, polyacrylates, docusate sodium, methylcellulose, agarchitosan, gums, guar gum, and mixtures thereof; preferably from sodium croscarmellose and crospovidone; more preferably sodium croscarmellose.
Clause 42: The pharmaceutical composition according to any of the preceding clauses, wherein the extragranular phase comprises a single extragranular disintegrant.
Clause 43: The pharmaceutical composition according to any of the preceding clauses, wherein the one or more extragranular disintegrants comprise or essentially consist of sodium croscarmellose.
Clause 44: The pharmaceutical composition according to any of the preceding clauses, wherein the total content of the one or more extragranular disintegrants is within the range of 5.5±4.0 wt.-%, preferably 5.5±3.5 wt.-%, more preferably 5.5±3.0 wt.-%, still more preferably 5.5±2.5 wt.-%, yet more preferably 5.5±2.0 wt.-%, even more preferably 5.5±1.5 wt.-%, most preferably 5.5±1.0 wt.-%, and in particular 5.5±0.5 wt.-%, in each case relative to the total weight of the pharmaceutical composition.
Clause 45: The pharmaceutical composition according to any of the preceding clauses, wherein the one or more extragranular lubricants are independently selected from fatty acids, fatty acid esters, fatty acid glyceride esters, metal salts of fatty acids, hydrogenated vegetable oil, hydrogenated castor oil, waxes, boric acid, sodium stearyl fumarate, macrogols, and sugar esters; preferably from magnesium stearate and sodium stearyl fumarate; more preferably magnesium stearate.
Clause 46: The pharmaceutical composition according to any of the preceding clauses, wherein the extragranular phase comprises a singly extragranular lubricant.
Clause 47: The pharmaceutical composition according to any of the preceding clauses, wherein the one or more extragranular lubricants comprise or essentially consist of magnesium stearate.
Clause 48: The pharmaceutical composition according to any of the preceding clauses, wherein the total content of the one or more extragranular lubricants is within the range of 0.9±0.8 wt.-%, preferably 0.9±0.7 wt.-%, more preferably 0.9±0.6 wt.-%, still more preferably 0.9±0.5 wt.-%, yet more preferably 0.9±0.4 wt.-%, even more preferably 0.9±0.3 wt.-%, most preferably 0.9±0.2 wt.-%, and in particular 0.9±0.1 wt.-%, in each case relative to the total weight of the pharmaceutical composition.
Clause 49: The pharmaceutical composition according to any of the preceding clauses, wherein the intragranular phase does not contain any intragranular lubricant.
Clause 50: The pharmaceutical composition according to any of the preceding clauses, wherein the overall content of the one or more intragranular diluents and the one or more extragranular diluents is at least 50 wt.-%, preferably at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%, in each case relative to the total weight of the pharmaceutical composition.
Clause 51: The pharmaceutical composition according to any of the preceding clauses, wherein the overall content of the one or more intragranular diluents and the one or more extragranular diluents is within the range of 85±10 wt.-%, preferably 85±9.0 wt.-%, more preferably 85±8.0 wt.-%, still more preferably 85±7.0 wt.-%, yet more preferably 85±6.0 wt.-%, even more preferably 85±5.0 wt.-%, most preferably 85±4.0 wt.-%, and in particular 85±3.0 wt.-%, in each case relative to the total weight of the pharmaceutical composition.
Clause 52: The pharmaceutical composition according to any of the preceding clauses, wherein the overall content of the one or more intragranular disintegrants and the one or more extragranular disintegrants is at least 2.0 wt.-%, preferably at least 2.5 wt.-%, more preferably at least 3.0 wt.-%, still more preferably at least 3.5 wt.-%, yet more preferably at least 4.0 wt.-%, even more preferably at least 4.5 wt.-%, most preferably at least 5.5 wt.-%, and in particular at least 6.0 wt.-%, in each case relative to the total weight of the pharmaceutical composition.
Clause 53: The pharmaceutical composition according to any of the preceding clauses, wherein the overall content of the one or more intragranular disintegrants and the one or more extragranular disintegrants is within the range of 6.9±2.4 wt.-%, preferably 6.9±2.1 wt.-%, more preferably 6.9±1.8 wt.-%, still more preferably 6.9±1.5 wt.-%, yet more preferably 6.9±1.2 wt.-%, even more preferably 6.9±0.9 wt.-%, most preferably 6.9±0.6 wt.-%, and in particular 6.9±0.3 wt.-%, in each case relative to the total weight of the pharmaceutical composition.
Clause 54: The pharmaceutical composition according to any of the preceding clauses, wherein the intragranular phase is wet granulated; preferably with a aqueous solvent; more preferably water.
Clause 55: The pharmaceutical composition according to any of the preceding clauses, wherein the total content of the intragranular phase is at least 7.5 wt.-%, preferably at least 10 wt.-%, more preferably at least 12.5 wt.-%, still more preferably at least 15 wt.-%, yet more preferably at least 17.5 wt.-%, even more preferably at least 20 wt.-%, most preferably at least 22.5 wt.-%, and in particular at least 6.0 wt.-%, in each case relative to the total weight of the pharmaceutical composition.
Clause 56: The pharmaceutical composition according to any of the preceding clauses, wherein the total content of the intragranular phase is within the range of 32±16 wt.-%, preferably 32±15 wt.-%, more preferably 32±14 wt.-%, still more preferably 32±13 wt.-%, yet more preferably 32±12 wt.-%, even more preferably 32±11 wt.-%, most preferably 32±10 wt.-%, and in particular 32±9.0 wt.-%, in each case relative to the total weight of the pharmaceutical composition.
Clause 57: The pharmaceutical composition according to any of the preceding clauses, wherein the total content of the extragranular phase is at least 40 wt.-%, preferably at least 42.5 wt.-%, more preferably at least 45 wt.-%, still more preferably at least 47.5 wt.-%, yet more preferably at least 50 wt.-%, even more preferably at least 52.5 wt.-%, most preferably at least 55 wt.-%, and in particular at least 57.5 wt.-%, in each case relative to the total weight of the pharmaceutical composition.
Clause 58: The pharmaceutical composition according to any of the preceding clauses, wherein the total content of the extragranular phase is within the range of 68±16 wt.-%, preferably 68±15 wt.-%, more preferably 68±14 wt.-%, still more preferably 68±13 wt.-%, yet more preferably 68±12 wt.-%, even more preferably 68±11 wt.-%, most preferably 68±10 wt.-%, and in particular 68±9.0 wt.-%, in each case relative to the total weight of the pharmaceutical composition.
Clause 59: The pharmaceutical composition according to any of the preceding clauses, which besides the ezetimibe, the pharmaceutically acceptable salt and/or solvate thereof and the pitavastatin, the pharmaceutically acceptable salt and/or solvate thereof does not contain any additional pharmacologically active ingredient.
Clause 60: An oral dosage form comprising or essentially consisting of the pharmaceutical composition according to any of the preceding clauses.
Clause 61: The dosage form according to clause 60, which is a tablet; preferably a single layer tablet.
Clause 62: The dosage form according to clause 61, which is not coated.
Clause 63: The dosage form according to clause 60, which is a capsule.
Clause 64: The dosage form according to any of clauses 60 to 63, which contains - the ezetimibe, the pharmaceutically acceptable salt and/or solvate thereof at a dosage of 2 mg or 4 mg; expressed as equivalent dose of the non-salt form of ezetimibe; and - the pitavastatin, the pharmaceutically acceptable salt and/or solvate thereof at a dosage of 10 mg; expressed as equivalent dose of the non-salt form of ezetimibe.
Clause 65: The dosage form according to any of clauses 60 to 64, which under *in vitro* conditions in accordance with USP paddle method (optionally equipped with 7-coil helical sinker), at 75 rpm and 37±0.5°C in 900 mL of phosphate buffer at pH 6.8 with 0.2% w/v Tween 80 has released after 5 minutes at least 50% of the content of ezetimibe that was originally contained in the dosage form; preferably at least 55%, more preferably at least 60%, still more preferably at least 65%, most preferably at least 70%, and in particular at least 75%.
Clause 66: The dosage form according to any of clauses 60 to 64, which under *in vitro* conditions in accordance with USP paddle method (optionally equipped with 7-coil helical sinker), at 75 rpm and 37±0.5°C in 900 mL of 0.005 M HC1 has released after 5 minutes at least 10% of the content of pitavastatin that was originally contained in the dosage form, preferably at least 15%, more preferably at least 20%, still more preferably at least 25%, most preferably at least 30%.
Clause 67: The dosage form according to any of clauses 60 to 66 for use in the treatment of hyperlipidemia and/or hypercholesterolemia (primary hypercholesterolemia [heterozygous familial and non-familial], mixed hyperlipidemia or homozygous familial hypercholesterolemia) or for reduction of cardiovascular event risk.
Clause 68: A process for the preparation of the pharmaceutical composition according to any of clauses 1 to 59 comprising the steps of A) preparing an intragranular phase comprising the sub-steps: a) dispersing one or more intragranular binders in a suitable liquid; preferably an aqueous liquid or an organic solvent; more preferably water; thereby obtaining a dispersion; b) homogenizing ezetimibe, a pharmaceutically acceptable salt and/or solvate thereof in the dispersion obtained in sub-step a) thereby obtaining a suspension; c) dispersing one or more intragranular surfactants in the dispersion obtained in step b) thereby obtaining a granulation liquid; d) spraying the granulation liquid onto a mixture comprising one or more intragranular diluents, optionally one or more intragranular disintegrants, and optionally one or more further pharmaceutically acceptable intragranular excipients, thereby obtaining granules; e) drying and sieving the granules obtained in sub-step d) thereby obtaining the intragranular phase; B) preparing an extragranular phase comprising the sub steps: f) homogenizing pitavastatin, a pharmaceutically acceptable salt and/or solvate thereof optionally together with one or more extragranular pharmaceutically acceptable excipients, preferably by means of a homogenizer, thereby obtaining a homogenous mixture; and g) adding one or more extragranular diluents and one or more extragranular disintegrants to the homogenous mixture obtained in sub-step f) thereby obtaining the extragranular phase; C) preparing a common blend comprising the sub-steps: h) blending the intragranular phase obtained in sub-step e) with the extragranular phase obtained in sub-step g); i) mixing one or more extragranular lubricants with one or more extragranular diluents to obtain a homogeneous mixture; and j) blending the mixtures obtained in sub steps h) and i) to obtain the pharmaceutical composition.
Clause 69: A solid pharmaceutical composition, which is obtainable by the process according to clause 68.
Clause 70: A process for the preparation of an oral dosage form according to any of clauses 60 to 67 comprising the process for the preparation of the pharmaceutical composition according to clause 68.
Clause 71: The process according to clause 70, which comprises the additional steps of D) compressing the pharmaceutical composition prepared in step C) into a tablet; or filling a capsule with the pharmaceutical composition prepared in step C); and E) optionally, film-coating the tablet obtained in step D). Clause 72: An oral dosage form, which is obtainable by the process according to clause 70 or 71.

The following examples further illustrate the invention but are not to be construed as limiting its scope.

### Comparative Example 1 - single layered tablet comprising two separate granulates:

Single layered tablets having the following composition were prepared:

| technological process | components | Content | |
|---|---|---|---|
| | | [mg] | [wt.-%] |
| granulation of ezetimibe in fluid bed granulator | ezetimibe | 10.00 | 3.33 |
| | mannitol pearlitol 160 C | 54.00 | 18.00 |
| | povidone K 30 | 3.00 | 1.00 |
| | sodium croscarmellose | 4.00 | 1.33 |
| | sodium lauryl sulfate | 2.00 | 0.67 |
| | purified water | q.s. | - |
| granulation of pitavastatin in fluid bed granulator | pitavastatin calcium salt | 4.18 | 1.39 |
| | HPMC 5CP/Methocel | 5.32 | 1.77 |
| | lactose crystalline 200 mesh | 100.00 | 33.33 |
| | purified water | q.s. | - |
| preparing a blend, mixing | lactose monohydrate (Tablettose 70) | 50.00 | 16.67 |
| | microcrystalline cellulose | 50.00 | 16.67 |
| | sodium croscarmellose | 15.00 | 5.00 |
| | magnesium stearate | 2.50 | 0.83 |
| total | | 300.00 | 100.00 |

Povidone (binder) was dissolved in purified water thereby obtaining an aqueous binder solution. Ezetimibe was homogenized in the aqueous binder solution thereby obtaining an aqueous suspension. Sodium lauryl sulfate (surfactant) was dispersed in the aqueous solution thereby obtaining a granulation liquid. The granulation liquid was sprayed onto a mixture of mannitol and sodium croscarmellose in a fluid bed granulator. The obtained granulate was dried and sieved.

Pitavastatin and hydroxypropyl methyl cellulose (HPMC) were homogenized in water to obtain a granulation liquid. The granulation liquid is sprayed onto lactose monohydrate. The obtained granulate was dried and sieved.

Granulates were combined to obtain a blend. To the blend lactose, microcrystalline cellulose, and sodium croscarmellose were added and mixed. To the obtained mixture a homogenized mixture of magnesium stearate and lactose was added and mixed to obtain a compression blend.

The compression blend was compressed into single layered tablets.

### Inventive Example 2 - single layered tablet comprising intragranular ezetimibe and extragranular pitavastatin:

Single layer tablets having the following composition were prepared:

| technological process | components [mg] | | [mg] | | | | | | | |
|---|---|---|---|---|---|---|---|---|---|---|
| | | | 2-1 | 2-2 | 2-3 | 2-4 | 2-5 | 2-6 | 2-7 | 2-8 |
| granulation of ezetimibe in fluid bed granulator | (i) | ezetimibe | 10.00 | 10.00 | 10.00 | 10.00 | 10.00 | 10.00 | 10.00 | 10.00 |
| | (ii) | mannitol pearlitol 160 c | 54.00 | 101.00 | 50.00 | / | / | / | / | / |
| | | lactose crystalline 200 mesh | / | / | / | 50.00 | 50.00 | 50.00 | 50.00 | 54.00 |
| | (iii) | povidone K 30 | 3.00 | 6.00 | 3.00 | 3.00 | 3.00 | 3.00 | 3.00 | 3.00 |
| | (iv) | sodium croscarmellose | 4.00 | 4.00 | 4.00 | 4.00 | 4.00 | 4.00 | 4.00 | 4.00 |
| | (v) | sodium lauryl sulfate | 2.00 | 2.00 | 6.00 | 6.00 | 6.00 | 6.00 | 6.00 | 2.00 |
| | purified water | | q.s. | q.s. | q.s. | q.s. | q.s. | q.s. | q.s. | q.s. |
| preparing a blend, mixing | (vi) | pitavastatin calcium salt | 4.18 | 4.18 | 4.18 | 4.18 | 4.18 | 2.09 | 2.09 | 4.18 |
| | (vii) | lactose monohydrate (Tablettose 80) | 105.32 | 105.32 | 105.32 | 105.32 | 105.32 | 107.41 | 107.41 | 88.32 |
| | | microcrystalline cellulose type 102 | 50.00 | 50.00 | 100.00 | 100.00 | 50.00 | 100.00 | 100.00 | 100.00 |
| | | lactose crystalline 200mesh | / | / | / | / | / | / | / | 17.00 |
| | (viii) | sodium croscarmellose | 15.00 | 15.00 | 15.00 | 15.00 | 15.00 | 15.00 | 15.00 | 15.00 |
| | (ix) | magnesium stearate | 2.50 | 2.50 | 2.50 | 2.50 | 2.50 | 2.50 | 2.50 | 2.50 |
| film coating | coating mixture | | / | / | / | / | / | 8.91 | / | / |
| | iron oxide, yellow | | / | / | / | / | / | 0.09 | / | / |
| | purified water | | / | / | / | / | / | q.s. | / | / |
| total | | | 250.00 | 300.00 | 300.00 | 300.00 | 250.00 | 309.00 | 300.00 | 300.00 |

| technological process | components [wt.-%] | | [wt.-%] | | | | | | | |
|---|---|---|---|---|---|---|---|---|---|---|
| | | | 2-1 | 2-2 | 2-3 | 2-4 | 2-5 | 2-6 | 2-7 | 2-8 |
| granulation of ezetimibe in fluid bed granulator | (i) | ezetimibe | 4.00 | 3.33 | 3.33 | 3.33 | 4.00 | 3.24 | 3.33 | 3.33 |
| | (ii) | mannitol pearlitol 160 c | 21.60 | 33.67 | 16.67 | / | / | / | / | / |
| | | lactose crystalline 200 mesh | / | / | / | 16.67 | 20.00 | 16.18 | 16.67 | 18.00 |
| | (iii) | povidone K 30 | 1.20 | 2.00 | 1.00 | 1.00 | 1.20 | 0.97 | 1.00 | 1.00 |
| | (iv) | sodium croscarmellose | 1.60 | 1.33 | 1.33 | 1.33 | 1.60 | 1.29 | 1.33 | 1.33 |
| | (v) | sodium lauryl sulfate | 0.80 | 0.67 | 2.00 | 2.00 | 2.40 | 1.94 | 2.00 | 0.67 |
| | purified water | | / | / | / | / | / | / | / | / |
| | *subtotal* | | *29.20* | *41.00* | *24.33* | *24.33* | *29.20* | 23.62 | *24.33* | *24.33* |
| preparing a blend, mixing | (vi) | pitavastatin calcium salt | 1.67 | 1.39 | 1.39 | 1.39 | 1.67 | 0.68 | 0.70 | 1.39 |
| | (vii) | lactose monohydrate (Tablettose 80) | 42.13 | 35.11 | 35.11 | 35.11 | 42.13 | 34.76 | 35.80 | 29.44 |
| | | microcrystalline cellulose type 102 | 20.00 | 16.67 | 33.33 | 33.33 | 20.00 | 32.36 | 33.33 | 33.33 |
| | | lactose crystalline 200 mesh | / | / | / | / | / | / | / | 5.67 |
| | (viii) | sodium croscarmellose | 6.00 | 5.00 | 5.00 | 5.00 | 6.00 | 4.85 | 5.00 | 5.00 |
| | (ix) | magnesium stearate | 1.00 | 0.83 | 0.83 | 0.83 | 1.00 | 0.81 | 0.83 | 0.83 |
| | *subtotal* | | *70.80* | *59.00* | 75.66 | 75.66 | *70.80* | *73.46* | 75.66 | 75.66 |
| film coating | coating mixture | | / | / | / | / | / | 2.88 | / | / |
| | iron oxide, yellow | | / | / | / | / | / | 0.03 | / | / |
| | purified water | | / | / | / | / | / | / | / | / |
| | *subtotal* | | / | / | / | / | / | 2.91 | / | / |
| total | | | 100.00 | 100.00 | 100.00 | 100.00 | 100.00 | 100.00 | 100.00 | 100.00 |

Povidone (binder) was dissolved in purified water thereby obtaining an aqueous binder solution. Ezetimibe was homogenized in the aqueous binder solution thereby obtaining an aqueous suspension. Sodium lauryl sulfate (surfactant) was dispersed in the aqueous solution thereby obtaining a granulation liquid. The granulation liquid was sprayed onto a mixture of mannitol or lactose and sodium croscarmellose in a fluid bed granulator. The obtained granulate is dried and sieved.

Pitavastatin was homogenized with lactose, then mixture of remaining diluents and sodium croscarmellose were added to the ezetimibe granulate obtained in step 1 and blended to obtain a blend. A homogenized mixture of magnesium stearate with lactose was added to the blend and mixed to obtain a compression blend.

The compression blend was compressed into single layered tablet.

### Comparative Example 3 - single layered tablet comprising intragranular pitavastatin and extragranular ezetimibe:

Single layer tablets having the following composition were prepared:

| technological process | components | Content | |
|---|---|---|---|
| | | [mg] | [wt.-%] |
| granulation of pitavastatin in fluid bed granulator | pitavastatin calcium salt | 4.18 | 1.39 |
| | HPMC 5CP/Methocel | 5.32 | 1.77 |
| | lactose crystalline 200 mesh | 100.00 | 33.33 |
| | purified water | q.s. | 0.00 |
| preparing a blend, mixing | ezetimibe | 10.00 | 3.33 |
| | sodium lauryl sulfate | 2.00 | 0.67 |
| | lactose monohydrate (Tablettose 80) | 111.00 | 37.00 |
| | microcrystalline cellulose type 102 | 50.00 | 16.67 |
| | sodium croscarmellose | 15.00 | 5.00 |
| | magnesium stearate | 2.50 | 0.83 |
| total | | 300.00 | 100.00 |

Pitavastatin and HPMC were homogenized in water to obtain a granulation liquid. The granulation liquid is sprayed onto lactose monohydrate. The obtained granulate is dried and sieved.

Ezetimibe, sodium lauryl sulfate, lactose monohydrate, sodium croscarmellose and microcrystalline cellulose were added to the pitavastatin granulate and mixed to obtain a blend. A homogenized mixture of magnesium stearate with lactose was added to the blend and mixed to obtain a compression blend.

The compression blend was compressed into single layered tablet.

### Comparative Example 4: single layered tablet comprising intragranular ezetimibe and intragranular pitavastatin:

Single layer tablets having the following composition were prepared:

| technological process | components | Content | |
|---|---|---|---|
| | | [mg] | [wt.-%] |
| granulation in fluid bed granulator | ezetimibe | 10.00 | 5.00 |
| | pitavastatin calcium salt | 4.30 | 2.15 |
| | sodium lauryl sulfate | 2.00 | 1.00 |
| | povidone K 30 | 2.66 | 1.33 |
| | lactose crystalline 200 mesh | 51.79 | 25.90 |
| | sodium croscarmellose | 4.00 | 2.00 |
| | purified water | q.s. | 0.00 |
| preparing a blend, mixing | lactose monohydrate (Tablettose 70) | 107.75 | 53.88 |
| | sodium croscarmellose | 15.00 | 7.50 |
| | magnesium stearate | 2.50 | 1.25 |
| total | | 200.00 | 100.00 |

Povidone (binder) was dissolved in purified water thereby obtaining an aqueous binder solution. Ezetimibe and pitavastatin were homogenized in the aqueous binder solution thereby obtaining an aqueous suspension. Sodium lauryl sulfate (surfactant) was dispersed in the aqueous solution thereby obtaining a granulation liquid. The granulation liquid was sprayed onto a mixture of lactose and sodium croscarmellose in a fluid bed granulator. The obtained granulate was dried and sieved.

Lactose and sodium croscarmellose were added to the ezetimibe/pitavastatin granulate obtained in step 1 and blended to obtain a blend. A homogenized mixture of magnesium stearate with lactose was added to the blend and mixed to obtain a compression blend.

The compression blend was compressed into single layered tablet.

### Example 5 - dissolution profiles:

*In vitro* dissolution of ezetimibe was measured under *in vitro* conditions in accordance with paddle method with side baskets 10 mesh at 50 rpm, peak vessel at 37±0.5°C in 900 mL. In acidic medium, 0.01 M HCl with 0.05 % Tween 80 and in basic medium ammonium phosphate buffer, pH 10.5 was used.

*In vitro* dissolution of pitavastatin was measured under *in vitro* conditions in accordance with USP baskets method at 50 rpm and 37±0.5°C in 900 mL of 0.005 M HC1.

### a) Ezetimibe

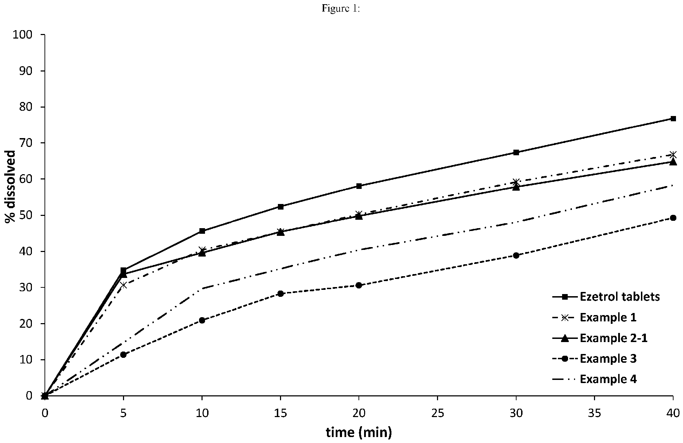

The *in vitro* dissolution profile for ezetimibe in basic medium (ammonium phosphate buffer, pH 10.5) released from the single layered tablets of Examples 1-4 in comparison to Ezetrol^{®} is shown in Figure 1.

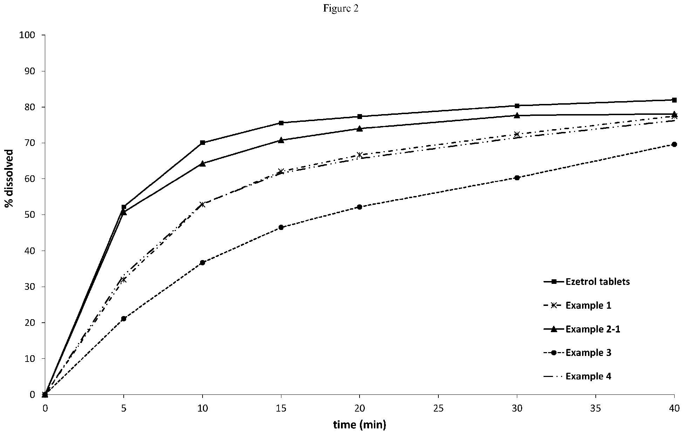

The *in vitro* dissolution profile for ezetimibe in acidic medium (0.01M HCl + 0.05% Tween 80) from the single layered tablets of Examples 1-4 in comparison to Ezetrol^{®} is shown in Figure 2.

As demonstrated by Figures 1 and 2, the *in vitro* dissolution profile of ezetimibe released from the single layered tablets of Inventive Examples 2 satisfy the similarity requirements compared to the *in vitro* dissolution profile of ezetimibe released from Ezetrol^{®} 10 mg (Batch No: T031622). In contrast, the compositions according to Comparative Examples 3 and 4 having intragranular pitavastatin do not provide *in vitro* dissolution profiles comparable to the *in vitro* dissolution profile released from Ezetrol^{®}. The composition of Comparative Example 1 comprising two separate granulates in a single layer tablet exhibits similar *in vitro* dissolution profile to Ezetrol^{®} in alkaline media (Figure 1), but this is not the case in the acidic media (Figure 2), where substantially slower *in vitro* dissolution profile is achieved. Thus, as demonstrated by Figures 1 and 2, the single layer tablets according to the invention provide an improved *in vitro* dissolution of ezetimibe compared to compositions known from the prior art.

### b) Pitavastatin

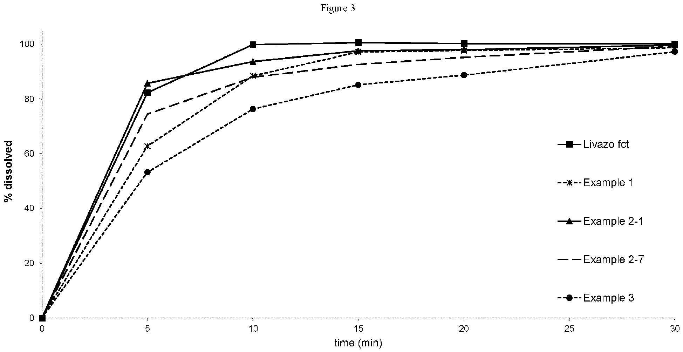

The *in vitro* dissolution profile for pitavastatin in acidic medium (0.005 M HC1) released from the single layered tablets of Examples 1-3 in comparison to Livazo^{®} is shown in Figure 3.

As demonstrated by Figure 3, the *in vitro* dissolution profile of pitavastatin released from the single layer tablet of Inventive Examples 2 satisfies the similarity requirements compared to the *in vitro* dissolution profile of pitavastatin released from Livazo^{®} 4 mg (Batch No: 4P00186), whereas the compositions according to Comparative Examples 1 and 3 show substantially slower *in vitro* dissolution profile. Thus, as demonstrated by Figure 3, the single layer tablets according to the invention provide an improved *in vitro* dissolution of pitavastatin compared to compositions known from the prior art documents.

### Example 6 - chemical stability:

Chemical stability of solid dosage forms according to the invention in comparison to the compositions prepared in accordance to the prior art documents was determined by accelerated stability test. The chemical stability of samples was tested under accelerated storage conditions (i.e. 50°C/ 75% r.h. for one month and 40°C/ 75% r.h for three months).

Accelerated stability results of solid dosage forms prepared according to Examples 1-4:

| condition | time | pitavastatin impurity lactone (%) | | | | ezetimibe impurity EBR1 (%) | | | |
|---|---|---|---|---|---|---|---|---|---|
| | | Ex. 1 | Ex. 2-1 | Ex. 3 | Ex. 4 | Ex. 1 | Ex. 2-1 | Ex. 3 | Ex. 4 |
| / | T₀ | 0.03 | 0.04 | 0.03 | 0.11 | 0.00 | 0.02 | 0.03 | 0.47 |
| 50°C/75% | 1 month | 0.24 | 0.30 | 0.20 | 1.55 | 0.02 | 0.00 | 0.03 | 0.49 |
| 40°C/75% | 3 months | 0.18 | 0.21 | 0.12 | 0.93 | 0.03 | 0.00 | 0.00 | 0.46 |

### HPLC method for determining content of pitavastatin and ezetimibe related substances:

In the analysis, liquid chromatograph coupled with a Diode Array Detector (DAD) was used.

### Chromatographic conditions:

Column: XSelect CSH C18, 150 mm x 4.6 mm, 3.5 µm particles
Mobile phase: gradient elution
mobile phase A = 0,01 M ammonium formate pH 3.30 ± 0.05
mobile phase B = acetonitrile

As demonstrated by the above data, chemical stability of ezetimibe and pitavastatin granulated together in the single layer tablet of Comparative Example 4 does not satisfy the stability requirements, this indicate that the two active ingredients cannot be incorporated together in the same part of the composition.

Stability of the compositions where the active ingredients are physically separated show comparable stability irrespective of the way of incorporation them into the composition.

### Example 7 - chemical stability and photostability:

### Accelerated stability results of solid dosage forms prepared according to Examples 2-6 (coated tablet) and 2-7 (uncoated tablet)

| condition | packaging | time | pitavastatin impurity lactone (%) | | ezetimibe impurity EBR1 (%) | |
|---|---|---|---|---|---|---|
| | | | Ex. 2-6 | Ex. 2-7 | Ex. 2-6 | Ex. 2-7 |
| / | / | T₀ | 0.08 | 0.10 | ≤ 0.10 | ≤ 0.10 |
| 50°C/75% | Alu/Alu | 1 month | 1.15 | 0.96 | ≤ 0.10 | ≤ 0.10 |
| 1200 kLux | Alu/Alu | / | / | 0.10 | / | ≤ 0.10 |
| 1200 kLux | PVC/PVDC/PVC | / | / | 0.09 | / | ≤ 0.10 |

As demonstrated by the above data, chemical stability of ezetimibe and pitavastatin incorporated together in the single layer tablet of Example 2-6 and in the film-coated tablet of Example 2-7 show that the film coating does not significantly improve the chemical stability of ezetimibe or pitavastatin.

Even the results of chemical stability of the single layer tablet of Example 2-7 after exposure to 1200 kLux show that the single layer tablet without film coating has comparable stability to the tablets, which were not exposed to light.

## Claims

1. A solid pharmaceutical composition comprising or essentially consisting of
- an intragranular phase comprising or essentially consisting of
(i) ezetimibe, a pharmaceutically acceptable salt and/or solvate thereof;
(ii) one or more intragranular diluents;
(iii) one or more intragranular binders;
(iv) optionally, one or more intragranular disintegrants; and
(v) optionally, one or more intragranular surfactants; and
- an extragranular phase comprising or essentially consisting of
(vi) pitavastatin, a pharmaceutically acceptable salt and/or solvate thereof;
(vii) one or more extragranular diluents;
(viii) optionally, one or more extragranular disintegrants; and
(ix) optionally, one or more extragranular lubricants.

2. The pharmaceutical composition according to claim 1, wherein the one or more intragranular diluents are independently selected from monosaccharides, disaccharides, oligosaccharides, and sugar alcohols; preferably from lactose and mannitol; more preferably from crystalline lactose and mannitol.

3. The pharmaceutical composition according to claim 1 or 2, wherein the total content of the one or more intragranular diluents is at least 2.0 wt.-%, preferably at least 4.0 wt.-%, more preferably at least 6.0 wt.-%, still more preferably at least 8.0 wt.-%, yet more preferably at least 10 wt.-%, even more preferably at least 12 wt.-%, most preferably at least 14 wt.-%, and in particular at least 16 wt.-%, in each case relative to the total weight of the pharmaceutical composition.

4. The pharmaceutical composition according to any of the preceding claims, wherein the one or more intragranular binders comprise or essentially consist of polyvinylpyrrolidone.

5. The pharmaceutical composition according to any of the preceding claims, wherein the one or more intragranular disintegrants comprise or essentially consist of sodium croscarmellose.

6. The pharmaceutical composition according to any of the preceding claims, wherein the one or more intragranular surfactants comprise or essentially consist of sodium lauryl sulfate or polysorbate 80.

7. The pharmaceutical composition according to any of the preceding claims, wherein the one or more extragranular diluents comprise or essentially consist of lactose and microcrystalline cellulose; more preferably lactose monohydrate and microcrystalline cellulose; or lactose monohydrate, crystalline lactose and microcrystalline cellulose.

8. The pharmaceutical composition according to any of the preceding claims, wherein the total content of the one or more extragranular diluents is at least 15 wt.-%, preferably at least 20 wt.-%, more preferably at least 25 wt.-%, still more preferably at least 30 wt.-%, yet more preferably at least 35 wt.-%, even more preferably at least 40 wt.-%, most preferably at least 45 wt.-%, and in particular at least 50 wt.-%, in each case relative to the total weight of the pharmaceutical composition.

9. The pharmaceutical composition according to any of the preceding claims, wherein the one or more extragranular disintegrants comprise or essentially consist of sodium croscarmellose.

10. The pharmaceutical composition according to any of the preceding claims, wherein the one or more extragranular lubricants comprise or essentially consist of magnesium stearate.

11. The pharmaceutical composition according to any of the preceding claims, wherein the overall content of the one or more intragranular diluents and the one or more extragranular diluents is at least 50 wt.-%, preferably at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%, in each case relative to the total weight of the pharmaceutical composition.

12. The pharmaceutical composition according to any of the preceding claims, wherein the overall content of the one or more intragranular disintegrants and the one or more extragranular disintegrants is at least 2.0 wt.-%, preferably at least 2.5 wt.-%, more preferably at least 3.0 wt.-%, still more preferably at least 3.5 wt.-%, yet more preferably at least 4.0 wt.-%, even more preferably at least 4.5 wt.-%, most preferably at least 5.5 wt.-%, and in particular at least 6.0 wt.-%, in each case relative to the total weight of the pharmaceutical composition.

13. The pharmaceutical composition according to any of the preceding claims, wherein the total content of the intragranular phase is at least 7.5 wt.-%, preferably at least 10 wt.-%, more preferably at least 12.5 wt.-%, still more preferably at least 15 wt.-%, yet more preferably at least 17.5 wt.-%, even more preferably at least 20 wt.-%, most preferably at least 22.5 wt.-%, and in particular at least 6.0 wt.-%, in each case relative to the total weight of the pharmaceutical composition.

14. The pharmaceutical composition according to any of the preceding claims, wherein the total content of the extragranular phase is at least 40 wt.-%, preferably at least 42.5 wt.-%, more preferably at least 45 wt.-%, still more preferably at least 47.5 wt.-%, yet more preferably at least 50 wt.-%, even more preferably at least 52.5 wt.-%, most preferably at least 55 wt.-%, and in particular at least 57.5 wt.-%, in each case relative to the total weight of the pharmaceutical composition.

15. An oral dosage form comprising or essentially consisting of the pharmaceutical composition according to any of the preceding claims; preferably a capsule or a tablet; more preferably a single layer tablet; still more preferably an uncoated single layer tablet.

## Patentansprüche

1. Eine feste pharmazeutische Zusammensetzung, umfassend oder im Wesentlichen bestehend aus
- einer intragranulären Phase, umfassend oder im Wesentlichen bestehend aus
(i) Ezetimib, einem pharmazeutisch annehmbaren Salz und/oder Solvat davon,
(ii) einem oder mehreren intragranulären Verdünnungsmitteln;
(iii) einem oder mehreren intragranulären Bindemitteln;
(iv) gegebenenfalls einem oder mehreren intragranulären Sprengmitteln; und
(v) gegebenenfalls einem oder mehreren intragranulären Tensiden; und
- eine extragranuläre Phase, umfassend oder im Wesentlichen bestehend aus
(vi) Pitavastatin, einem pharmazeutisch annehmbaren Salz und/oder Solvat davon,
(vii) einem oder mehreren extragranulären Verdünnungsmitteln;
(viii) gegebenenfalls einem oder mehreren extragranulären Sprengmitteln; und
(ix) gegebenenfalls einem oder mehreren extragranulären Gleitmitteln.

2. Die pharmazeutische Zusammensetzung nach Anspruch 1, wobei das eine oder die mehreren intragranulären Verdünnungsmittel unabhängig voneinander ausgewählt sind aus Monosacchariden, Disacchariden, Oligosacchariden und Zuckeralkoholen, vorzugsweise aus Lactose und Mannitol, noch bevorzugter aus kristalliner Lactose und Mannitol.

3. Die pharmazeutische Zusammensetzung nach Anspruch 1 oder 2, wobei der Gesamtgehalt des einen oder der mehreren intragranulären Verdünnungsmittel mindestens 2,0 Gew.-%, vorzugsweise mindestens 4,0 Gew.-%, stärker bevorzugt mindestens 6,0 Gew.-%, noch stärker bevorzugt mindestens 8,0 Gew.-%, noch stärker bevorzugt mindestens 10 Gew.-%, noch stärker bevorzugt mindestens 12 Gew.-%, am meisten bevorzugt mindestens 14 Gew.-% und insbesondere mindestens 16 Gew.-%, jeweils bezogen auf das Gesamtgewicht der pharmazeutischen Zusammensetzung, beträgt.

4. Die pharmazeutische Zusammensetzung nach einem der vorhergehenden Ansprüche, wobei das eine oder die mehreren intragranulären Bindemittel Polyvinylpyrrolidon umfassen oder im Wesentlichen daraus bestehen.

5. Die pharmazeutische Zusammensetzung nach einem der vorangehenden Ansprüche, wobei das eine oder die mehreren intragranulären Sprengmittel Natriumcroscarmellose umfassen oder im Wesentlichen daraus bestehen.

6. Die pharmazeutische Zusammensetzung nach einem der vorangehenden Ansprüche, wobei das eine oder die mehreren intragranulären Tenside Natriumlaurylsulfat oder Polysorbat 80 umfassen oder im Wesentlichen daraus bestehen.

7. Die pharmazeutische Zusammensetzung nach einem der vorangehenden Ansprüche, wobei das eine oder die mehreren extragranulären Verdünnungsmittel Lactose und mikrokristalline Cellulose, vorzugsweise Lactosemonohydrat und mikrokristalline Cellulose, oder Lactosemonohydrat, kristalline Lactose und mikrokristalline Cellulose umfassen oder im Wesentlichen daraus bestehen.

8. Die pharmazeutische Zusammensetzung nach einem der vorhergehenden Ansprüche, wobei der Gesamtgehalt des einen oder der mehreren extragranulären Verdünnungsmittel mindestens 15 Gew.-%, vorzugsweise mindestens 20 Gew.-%, noch bevorzugter mindestens 25 Gew.-%, noch bevorzugter mindestens 30 Gew.-%, noch bevorzugter mindestens 35 Gew.-%, noch bevorzugter mindestens 40 Gew.-%, am meisten bevorzugt mindestens 45 Gew.-% und insbesondere mindestens 50 Gew.-% beträgt, jeweils bezogen auf das Gesamtgewicht der pharmazeutischen Zusammensetzung.

9. Die pharmazeutische Zusammensetzung nach einem der vorhergehenden Ansprüche, wobei das eine oder die mehreren extragranulären Sprengmittel Natriumcroscarmellose umfassen oder im Wesentlichen daraus bestehen.

10. Die pharmazeutische Zusammensetzung nach einem der vorangehenden Ansprüche, wobei das eine oder die mehreren extragranulären Gleitmittel Magnesiumstearat umfassen oder im Wesentlichen daraus bestehen.

11. Die pharmazeutische Zusammensetzung nach einem der vorangehenden Ansprüche, wobei der Gesamtgehalt des einen oder der mehreren intragranulären Verdünnungsmittel und des einen oder der mehreren extragranulären Verdünnungsmittel mindestens 50 Gew.-%, vorzugsweise mindestens 55 Gew.-%, noch bevorzugter mindestens 60 Gew.-%, noch bevorzugter mindestens 65 Gew.-%, noch bevorzugter mindestens 70 Gew.-%, noch bevorzugter mindestens 75 Gew.-%, am meisten bevorzugt mindestens 80 Gew.-%, und insbesondere mindestens 85 Gew.-% beträgt, jeweils bezogen auf das Gesamtgewicht der pharmazeutischen Zusammensetzung.

12. Die pharmazeutische Zusammensetzung nach einem der vorhergehenden Ansprüche, wobei der Gesamtgehalt des einen oder der mehreren intragranulären Sprengmittel und des einen oder der mehreren extragranulären Sprengmittel mindestens 2,0 Gew.-%, vorzugsweise mindestens 2,5 Gew.-%, noch bevorzugter mindestens 3,0 Gew.-%, noch bevorzugter mindestens 3,5 Gew.-%, noch bevorzugter mindestens 4,0 Gew.-%, noch bevorzugter mindestens 4,5 Gew.-%, am meisten bevorzugt mindestens 5,5 Gew.-% und insbesondere mindestens 6,0 Gew.-% beträgt, jeweils bezogen auf das Gesamtgewicht der pharmazeutischen Zusammensetzung.

13. Die pharmazeutische Zusammensetzung nach einem der vorhergehenden Ansprüche, wobei der Gesamtgehalt der intragranulären Phase mindestens 7,5 Gew.-%, vorzugsweise mindestens 10 Gew.-%, noch bevorzugter mindestens 12,5 Gew.-%, noch bevorzugter mindestens 15 Gew.-%, noch bevorzugter mindestens 17,5 Gew.-%, noch bevorzugter mindestens 20 Gew.-%, am meisten bevorzugt mindestens 22,5 Gew.-% und insbesondere mindestens 6,0 Gew.-%, beträgt, jeweils bezogen auf das Gesamtgewicht der pharmazeutischen Zusammensetzung.

14. Die pharmazeutische Zusammensetzung nach einem der vorhergehenden Ansprüche, wobei der Gesamtgehalt der extragranulären Phase mindestens 40 Gew.-%, vorzugsweise mindestens 42,5 Gew.-%, noch bevorzugter mindestens 45 Gew.-%, noch bevorzugter mindestens 47,5 Gew.-%, noch bevorzugter mindestens 50 Gew.-%, noch bevorzugter mindestens 52,5 Gew.-%, am meisten bevorzugt mindestens 55 Gew.-% und insbesondere mindestens 57,5 Gew.-% beträgt, jeweils bezogen auf das Gesamtgewicht der pharmazeutischen Zusammensetzung.

15. Eine orale Darreichungsform, umfassend oder im wesentlichen bestehend aus der pharmazeutischen Zusammensetzung nach einem der vorhergehenden Ansprüche; vorzugsweise eine Kapsel oder eine Tablette; noch bevorzugter eine Einschichttablette; noch bevorzugter eine unbeschichtete Einschichttablette.

## Revendications

1. Composition pharmaceutique solide comprenant ou constituée essentiellement
- d'une phase intragranulaire comprenant ou constituée essentiellement
(i) de l'ézétimibe, d'un sel et/ou d'un solvate pharmaceutiquement acceptable de celui-ci ;
(ii) d'un ou plusieurs diluants intragranulaires ;
(iii) d'un ou plusieurs liants intragranulaires ;
(iv) éventuellement, d'un ou plusieurs désintégrants intragranulaires ; et
(v) éventuellement, d'un ou plusieurs tensioactifs intragranulaires ; et
- d'une phase extragranulaire comprenant ou constituée essentiellement
(vi) de la pitavastatine, d'un sel et/ou d'un solvate pharmaceutiquement acceptable de celle-ci ;
(vii) d'un ou plusieurs diluants extragranulaires ;
(viii) éventuellement, d'un ou plusieurs désintégrants extragranulaires ; et
(ix) éventuellement, d'un ou plusieurs lubrifiants extragranulaires.

2. Composition pharmaceutique selon la revendication 1, dans laquelle les un ou plusieurs diluants intragranulaires sont indépendamment choisis parmi les monosaccharides, les disaccharides, les oligosaccharides et les alcools de sucre ; de préférence parmi le lactose et le mannitol ; de manière davantage préférée parmi le lactose et le mannitol cristallins.

3. Composition pharmaceutique selon la revendication 1 ou 2, dans laquelle la teneur totale des un ou plusieurs diluants intragranulaires est d'au moins 2,0 % en poids, de préférence d'au moins 4,0 % en poids, de manière davantage préférée d'au moins 6,0 % en poids, de manière encore davantage préférée d'au moins 8,0 % en poids, de manière encore plus davantage préférée d'au moins 10 % en poids, de manière encore plus davantage préférée d'au moins 12 % en poids, de manière préférée entre toutes d'au moins 14 % en poids, et en particulier d'au moins 16 % en poids, dans chaque cas par rapport au poids total de la composition pharmaceutique.

4. Composition pharmaceutique selon l'une quelconque des revendications précédentes, dans laquelle les un ou plusieurs liants intragranulaires comprennent ou sont essentiellement constitués de polyvinylpyrrolidone.

5. Composition pharmaceutique selon l'une quelconque des revendications précédentes, dans laquelle les un ou plusieurs désintégrants intragranulaires comprennent ou sont essentiellement constitués de croscarmellose sodique.

6. Composition pharmaceutique selon l'une quelconque des revendications précédentes, dans laquelle les un ou plusieurs tensioactifs intragranulaires comprennent ou sont essentiellement constitués de laurylsulfate de sodium ou de polysorbate 80.

7. Composition pharmaceutique selon l'une quelconque des revendications précédentes, dans laquelle les un ou plusieurs diluants extragranulaires comprennent ou sont essentiellement constitués de lactose et de cellulose microcristalline ; de manière davantage préférée de lactose monohydraté et de cellulose microcristalline ; ou de lactose monohydraté, de lactose cristallin et de cellulose microcristalline.

8. Composition pharmaceutique selon l'une quelconque des revendications précédentes, dans laquelle la teneur totale des un ou plusieurs diluants extragranulaires est d'au moins 15 % en poids, de préférence d'au moins 20 % en poids, de manière davantage préférée d'au moins 25 % en poids, de manière encore davantage préférée d'au moins 30 % en poids, de manière encore plus davantage préférée d'au moins 35 % en poids, de manière encore plus davantage préférée d'au moins 40 % en poids, de manière préférée entre toutes d'au moins 45 % en poids, et en particulier d'au moins 50 % en poids, dans chaque cas par rapport au poids total de la composition pharmaceutique.

9. Composition pharmaceutique selon l'une quelconque des revendications précédentes, dans laquelle les un ou plusieurs désintégrants extragranulaires comprennent ou sont essentiellement constitués de croscarmellose sodique.

10. Composition pharmaceutique selon l'une quelconque des revendications précédentes, dans laquelle les un ou plusieurs lubrifiants extragranulaires comprennent ou sont essentiellement constitués de stéarate de magnésium.

11. Composition pharmaceutique selon l'une quelconque des revendications précédentes, dans laquelle la teneur globale des un ou plusieurs diluants intragranulaires et des un ou plusieurs diluants extragranulaires est d'au moins 50 % en poids, de préférence d'au moins 55 % en poids, de manière davantage préférée d'au moins 60 % en poids, de manière encore davantage préférée d'au moins 65 % en poids, de manière encore plus davantage préférée d'au moins 70 % en poids, de manière encore plus davantage préférée d'au moins 75 % en poids, de manière préférée entre toutes d'au moins 80 % en poids, et en particulier d'au moins 85 % en poids, dans chaque cas par rapport au poids total de la composition pharmaceutique.

12. Composition pharmaceutique selon l'une quelconque des revendications précédentes, dans laquelle la teneur globale des un ou plusieurs désintégrants intragranulaires et des un ou plusieurs désintégrants extragranulaires est d'au moins 2,0 % en poids, de préférence d'au moins 2,5 % en poids, de manière davantage préférée d'au moins 3,0 % en poids, de manière encore davantage préférée d'au moins 3,5 % en poids, de manière encore plus davantage préférée d'au moins 4,0 % en poids, de manière encore plus davantage préférée d'au moins 4,5 % en poids, de manière préférée entre toutes d'au moins 5,5 % en poids, et en particulier d'au moins 6,0 % en poids, dans chaque cas par rapport au poids total de la composition pharmaceutique.

13. Composition pharmaceutique selon l'une quelconque des revendications précédentes, dans laquelle la teneur totale de la phase intragranulaire est d'au moins 7,5 % en poids, de préférence d'au moins 10 % en poids, de manière davantage préférée d'au moins 12,5 % en poids, de manière encore plus davantage préférée d'au moins 15 % en poids, de manière encore plus davantage préférée d'au moins 17,5 % en poids, de manière encore plus davantage préférée d'au moins 20 % en poids, de manière préférée entre toutes d'au moins 22,5 % en poids, et en particulier d'au moins 6,0 % en poids, dans chaque cas par rapport au poids total de la composition pharmaceutique.

14. Composition pharmaceutique selon l'une quelconque des revendications précédentes, dans laquelle la teneur totale de la phase extragranulaire est d'au moins 40 % en poids, de préférence d'au moins 42,5 % en poids, de manière davantage préférée d'au moins 45 % en poids, de manière encore plus davantage préférée d'au moins 47,5 % en poids, de manière encore plus davantage préférée d'au moins 50 % en poids, de manière encore plus davantage préférée d'au moins 52,5 % en poids, de manière préférée entre toutes d'au moins 55 % en poids, et en particulier d'au moins 57,5 % en poids, dans chaque cas par rapport au poids total de la composition pharmaceutique.

15. Forme posologique orale comprenant ou constituée essentiellement de la composition pharmaceutique selon l'une quelconque des revendications précédentes ; de préférence une capsule ou un comprimé ; de manière davantage préférée un comprimé monocouche ; de manière encore davantage préférée un comprimé monocouche non enrobé.
